# EUROPÄISCHE PATENTSCHRIFT

(11) **EP 1 828 228 B1**
(45) Veröffentlichungstag und Bekanntmachung des Hinweises auf die Patenterteilung: **07.05.2008**
(21) Anmeldenummer: 05819403.6
(22) Anmeldetag: 13.12.2005
(51) Int. Cl.: C07K 7/56, C12P 21/06

(54) **VERFAHREN ZUR DEACYLIERUNG VON LIPOPEPTIDEN**
METHOD FOR THE DEACYLATION OF LIPOPEPTIDES
PROCEDE DE DESACYLATION DE LIPOPEPTIDES

(30) Priorität: 15.12.2004 DE 102004060750
(43) Veröffentlichungstag der Anmeldung: 05.09.2007
(73) Patentinhaber: Sanofi-Aventis Deutschland GmbH, 65929 Frankfurt am Main (DE)
(72) Erfinder: EHLERS, Eberhard, 65719 Hofheim (DE); DECKER, Heinrich, 65817 Eppstein (DE); RISSOM, Sebastian, 92340 Bourg la Reine (FR); SEIDEL, Guido, 60487 Frankfurt am Main (DE); OLLIGER, Reiner, 63486 Bruchköbel (DE)
(86) Internationale Anmeldenummer: PCT/EP2005/013336
(87) Internationale Veröffentlichungsnummer: WO 2006/063783

(56) Entgegenhaltungen:
- EP-A- 0 460 882
- L D BOECK ET AL.: "Deacylation of A21978C, an acidic lipopeptide antibiotic complex, by actinoplanes utahensis" JOURNAL OF ANTIBIOTICS., Bd. 41, Nr. 8, August 1988 (1988-08), Seiten 1085-1092, XP001028754 JP JAPAN ANTIBIOTICS RESEARCH ASSOCIATION, TOKYO.

## Beschreibung

Lipopeptide sind Cyclohexapeptide, die fermentativ gewonnen werden können und eine systemisch antifungische Aktivität besitzen, wobei sie die (1,3)-β-D-Glucan-Synthase, ein Schlüsselenzym der Zellwandbiosynthese von Pilzen hemmen. Ferner haben Lipopeptide antibakterielle Aktivität (Exp. Opin. Invest Drugs 2000, 9, 1797-1813). Es ist bekannt, dass Lipopeptide ungünstige physikalisch-chemische Eigenschaften wie Schwerlöslichkeit in Wasser oder eine Instabilität in alkalischer Lösung besitzen. Darüber hinaus zeigen die natürlichen Lipopeptide schwere Nebenwirkungen wie Schädigungen des Venenendothels, Zerstörung und Entzündungen von Geweben oder lokale toxische Effekte am Applikationsort.

Es besteht daher die Notwendigkeit zur Synthese neuer Lipopeptide mit verbesserten pharmakokinetischen und chemotherapeutischen Eigenschaften sowie geringerer Toxizität. Solche semisynthetischen Modifizierungen bestehen im allgemeinen in der Einführung saurer oder basischer Gruppen in das Molekülgerüst und im Ersatz der natürlichen aliphatischen Seitenkettensäure durch aromatische Acylkomponenten. Insbesondere der Abwandlung der Fettsäureseitenkette (N-Acylseitenkette) kommt zentrale Bedeutung bei Partialsynthesen von Lipopeptiden zu. Dies geschieht im allgemeinen derart, dass die N-Acyl-Seitenkette von Lipopeptiden mit einer natürlichen, Zell-assoziierten oder rekombinant erzeugten Deacylase breiter Substratspezifität auf enzymatischem Wege abgespalten wird und der resultierende Peptid-Zyklus, der sogenannte Nukleus, mit einer modifizierten aktivierten Säure re-acyliert wird (beispielsweise EP 1 189 932; EP 1 189 933).

Die europäische Patentanmeldung EP 0 460 882 beschreibt die Deacylierung der lipidischen Acylposition von Lipopeptiden mittels aufgereinigter Echinocandin B Decyclase. Das Enzym wird durch Fermentation von *Actinoplanes utahensis* (NRRL12052) produziert, wobei es nach der Fermentation zellassoziiert vorliegt, und zunächst mittels Salzbehandlung von den Zellen gelöst wird, bevor das so erhaltene gelöste Enzym in einem achtstufigen Verfahren aufgereinigt wird.

Im allgemeinen erfolgt die enzymatische Seitenkettenabspaltung aus Lipopeptiden dadurch, dass man das gereinigte Lipopeptid mit einer Kultur oder einem Kulturüberstand eines Mikroorganismus versetzt, der eine Deacylase breiter Substratspezifität produziert. Dies kann ein natürlicher *Actinoplanes utahensis-*Stamm (z.B. NRRL 12052; WO00/75177 und WO00/75178) oder ein rekombinanter Deacylaseproduzent sein, wie z.B. ein rekombinant veränderter *Streptomyces lividans*-Stamm. Eine weitere Verfahrensalternative ist, zur Lösung oder Suspension des gereinigten Lipopeptids eine gereinigte Deacylase hinzufügen. Nach erfolgter Seitenkettenabspaltung wird der Reaktionsüberstand von unlöslichen Bestandteilen befreit und der im Filtrat enthaltene wasserlösliche Nukleus aufgereinigt. Diese Vorgehensweise unter Verwendung gereinigter oder partiell gereinigter Substrate ist umständlich und großtechnisch nicht anwendbar.

J. Antibiotics XLI(8), Ss. 1085-1091 (1988) lehrt ein Verfahren zur Deacylierung von Lipopeptiden in unreinen Zusammensetzungen.

Die vorliegende Erfindung betrifft ein Verfahren zur enzymatischen Abspaltung der N-Acyl-Seitenkette aus Lipopeptiden unter Bildung des entsprechenden Nukleus, wobei
(a) das Lipopeptid fermentativ hergestellt wird, wobei das Lipopeptid an der Biomasse zellgebunden vorliegt, und die Biomasse mit dem anhaftenden Lipopeptid abgetrennt wird,
(b) die Biomasse des Fermentationsschrittes (a) mit dem anhaftenden Lipopeptid in einem wässrigen System re-suspendiert wird,
(c) eine geeignete Deacylase in gelöster oder fester Form zu der Suspension der Biomasse aus Schritt (b) gegeben wird, und sich der entsprechende Nukleus bildet, und
(d) der Nukleus optional isoliert und gereinigt wird,
dadurch gekennzeichnet, dass das in Schritt (a) fermentativ gewonnene Lipopeptid nach Fermentationsende als zellgebundene Biomasse ohne weitere Reinigung in Schritt (c) direkt mit einer Deacylase zur Reaktion gebracht wird, wodurch die über eine Amidbindung verknüpfte N-Acylkette abgespalten wird.

Optional wird in einem weiteren Schritt (e) der Nukleus mit einem Säurederivat zu einem semi-synthetischen Lipopeptid der Formel umgesetzt, wobei die dabei entstehende Amidfunktion durch eine Gruppe Phenyl-(C₅-C₈)Heteroaryl-Phenyl, C(O)-Biphenyl oder C(O)-Terphenyl substituiert ist.

Die vorliegende Erfindung betrifft ferner ein Verfahren zur enzymatischen Abspaltung der N-Acyl-Seitenkette R₁₁ aus Lipopeptiden der Formel (I) 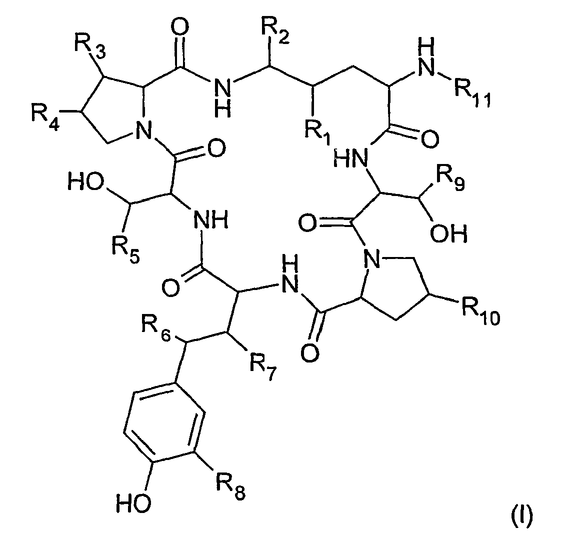 wobei
R₁ = H, OH oder NRₓR_{y} ist, wobei
Rₓ und R_{y} unabhängig voneinander H oder (C₁-C₆)Alkyl sind,
R₂ = H, OH, NH(CH₂)₂NH₂,
R₃ = H, OH,
R₄ = H, Me, NH₂, -NH-C(=NH)NH₂,
R₅ = H, Me, CH₂-C(=O)NH₂, CH₂CH₂NH₂,
R₆ = H, OH,
R₇ = H, OH,
R₈ = H, OSO₃H, OSO₃Na, NH-C(=O)CH₂NH₂,
R₉ = Me, und
R₁₀ = H, OH,
R₁₁ = eine Gruppe C(O)-(C₆-C₂₄)Alkyl, eine Gruppe C(O)-(C₆-C₂₄)Alkenyl, eine Gruppe C(O)-(C₆-C₂₄)Alkadienyl, oder eine Gruppe C(O)-(C₆-C₂₄)Alkatrienyl,
unter Bildung des entsprechenden Nukleus der Formel (II) 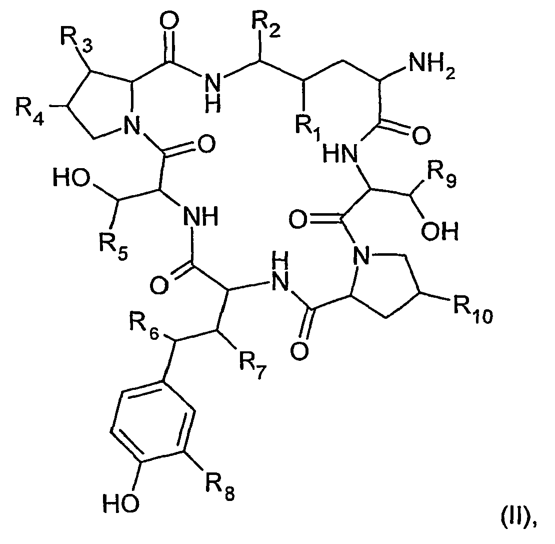 wobei R₁-R₁₀ die für Formel (I) genannte Bedeutung haben, und wobei sofern vorhanden Alkyl-, Alkenyl-, Alkadienyl- und Alkatrienylgruppen in Verbindungen der Formel (I) und (II) verzweigt oder geradkettig sein können,
wobei
(a) das Lipopeptid der Formel (I) fermentativ hergestellt wird, wobei das Lipopeptid an der Biomasse zellgebunden vorliegt, und die Biomasse mit dem anhaftenden Lipopeptid abgetrennt wird,
(b) die Biomasse des Fermentationsschrittes (a) mit dem anhaftenden Lipopeptid in einem wässrigen System re-suspendiert wird,
(c) eine geeignete Deacylase in gelöster oder fester Form zu der Suspension der Biomasse aus Schritt (b) gegeben wird, und sich der entsprechende Nukleus der Formel (II) bildet, und
(d) der Nukleus optional isoliert und gereinigt wird,
dadurch gekennzeichnet, dass das in Schritt (a) fermentativ gewonnene Lipopeptid nach Fermentationsende als zellgebundene Biomasse ohne weitere Reinigung in Schritt (c) direkt mit einer Deacylase zur Reaktion gebracht wird, wodurch die über eine Amidbindung verknüpfte N-Acylkette abgespalten wird.

Die vorliegende Erfindung betrifft ferner ein Verfahren zur enzymatischen Abspaltung der N-Acyl-Seitenkette R₂₀ aus Lipopeptiden der Formel (III) 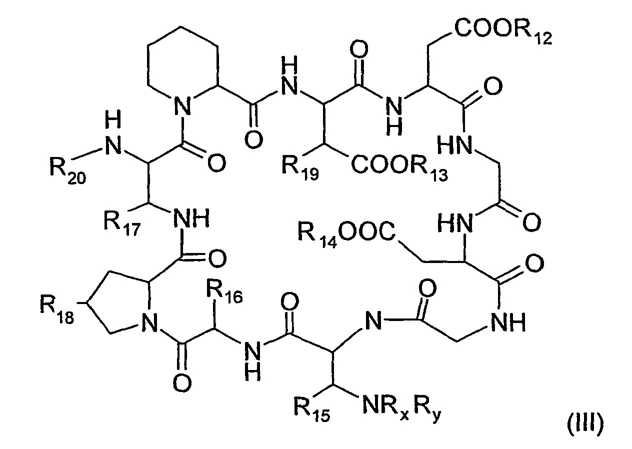 wobei
- R₁₂, R₁₃, R₁₄, R₁₅, R₁₆, R₁₇, R₁₈, R₁₉, Rₓ und R_{y}: unabhängig voneinander H oder (C₁-C₆)Alkyl sind, und
- R₂₀ =: eine Gruppe C(O)-CH(CH₂COR₂₁)-NH-CO-(C₆-C₂₄)Alkyl,
eine Gruppe C(O)-CH(CH₂COR₂₁)-NH-CO-(C₆-C₂₄)Alkenyl, eine Gruppe C(O)-CH(CH₂COR₂₁)-NH-CO-(C₆-C₂₄)Alkadienyl, oder eine Gruppe C(O)-CH(CH₂COR₂₁)-NH-CO-(C₆-C₂₄)Alkatrienyl, wobei R₂₁ OH oder NH₂ ist,
unter Bildung des entsprechenden Nukleus der Formel (IV) 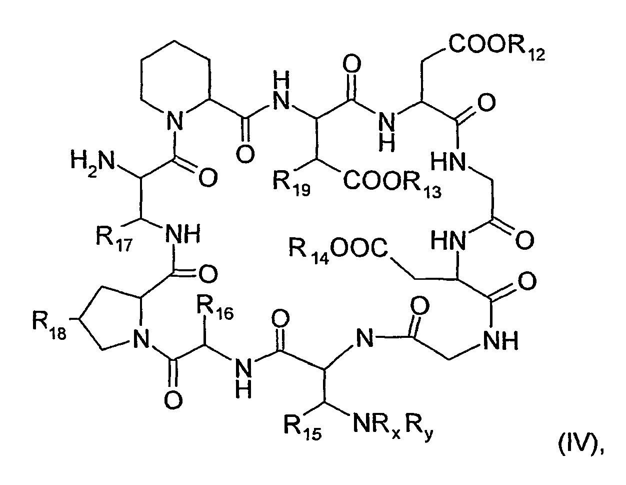 wobei R₁₂, R₁₃, R₁₄, R₁₅, R₁₆, R₁₇, R₁₈, R₁₉, Rₓ und R_{y} die für Formel (III) genannte Bedeutung haben, und wobei Alkyl-, Alkenyl-, Alkadienyl- und Alkatrienylgruppen sofern vorhanden in Verbindungen der Formel (IIII) und (IV) verzweigt oder geradketttig sein können,
wobei
(a) das Lipopeptid der Formel (III) fermentativ hergestellt wird, wobei das Lipopeptid an der Biomasse zellgebunden vorliegt, und die Biomasse mit dem anhaftenden Lipopeptid abgetrennt wird,
(b) die Biomasse des Fermentationsschrittes (a) mit dem anhaftenden Lipopeptid in einem wässrigen System re-suspendiert wird,
(c) eine geeignete Deacylase in gelöster oder fester Form zu der Suspension der Biomasse aus Schritt (b) gegeben wird, und sich der entsprechende Nukleus der Formel (IV) bildet, und
(d) der Nukleus optional isoliert und gereinigt wird,
dadurch gekennzeichnet, dass das in Schritt (a) fermentativ gewonnene Lipopeptid nach Fermentationsende als zellgebundene Biomasse ohne weitere Reinigung in Schritt (c) direkt mit einer Deacylase zur Reaktion gebracht wird, wodurch die über eine Amidbindung verknüpfte N-Acylkette abgespalten wird.

Optional wird in einem weiteren Schritt (e) der Nukleus (IV) mit einem Säurederivat zu einem semi-synthetischen Lipopeptid der Formel (III') umgesetzt wird, in dem die Gruppe R₂₀ definiert ist als eine Gruppe C(O)-Phenyl-(C₅-C₈)Heteroaryl-Phenyl, C(O)-Biphenyl oder C(O)-Terphenyl.

(C₁-C₆)Alkyl bedeutet ein Kohlenwasserstoffrest mit 1, 2, 3, 4, 5 oder 6 C-Atomen. Beispiele für (C₁-C₆)Alkylreste sind Methyl, Ethyl, n-Propyl, Isopropyl (1-Methylethyl), n-Butyl, Isobutyl (2-Methylpropyl), sec-Butyl (1-Methylpropyl), tert-Butyl (1,1-Dimethylethyl), n-Pentyl, Isopentyl, tert-Pentyl, Neopentyl, Hexyl.

(C₆-C₂₄)Alkyl bedeutet entsprechend ein Kohlenwasserstoffrest mit 6 bis 24 C-Atomen. Alkylreste können geradkettig oder verzweigt sein. Als (C₆-C₂₄)Alkylreste sind Fettsäurereste bevorzugt, beispielsweise Hexyl, Octyl, Decanyl, Undecanyl, Dodecanyl, Tridecanyl, Tetradecanyl (Myristyl), Pentadecanyl, Hexadecanyl, Heptadecanyl, Octadecanyl (Stearyl), Nonadecanyl, Eicosanyl, Dicosanyl, 9,11-Dimethyl-tridecanyl, 11-Methyl-tridecanyl.

Die vorherige Isolierung und aufwendige Aufreinigung des Lipopeptid-Ausgangsmaterials entfällt bei dieser Vorgehensweise, da das an die Biomasse gebundene Lipopeptid direkt im Deacylierungsschritt eingesetzt wird. Das erfindungsgemäße Verfahren ist daher für den großtechnischen Maßstab geeignet. Verunreinigungen und Nebenprodukte, die sich in der komplexen Nährlösung des Verfahrensschritts (a) befinden, werden auf einfache Weise, z.B. durch Filtration oder Zentrifugieren, entfernt, bevor in Verfahrensschritt (b) die Deacylierung erfolgt. Verunreinigungen und Nebenprodukte sind beispielsweise Medienbestandteile, Stoffwechselprodukte des Stammes oder Enzyme.

Optional wird die in Verfahrensschritt (a) gewonnene Biomasse mit dem anhaftenden Lipopeptid vor dem Re-suspendieren in Verfahrensschritt (b) mit Wasser gewaschen.

Das wässrige System zum Re-suspendieren des zellgebundenen Lipopeptids in Schritt (b) des erfindungsgemäßen Verfahrens ist Wasser oder eine wässrige Pufferlösung, vorzugsweise mit einen pH-Wert von 7,2 bis 4,5, besonders bevorzugt von 6,0 bis 5,0, speziell bevorzugt bei einem pH-Wert von 5,5. Als Pufferlösungen können alle dem Fachmann bekannte Lösungen verwendet werden. Die anschließende Deacylierung kann im pH-Bereich von pH 4 bis 9 durchgeführt werden; bevorzugt ist ein Bereich von pH 4,6 bis pH 7,8 mit einem Wirkungsoptimum des Enzyms bei pH 6-6,2.

Das erfindungsgemäße Verfahren betrifft vorzugsweise Verbindungen der Formel (I), in denen R₁₁ 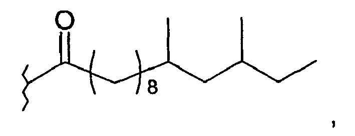 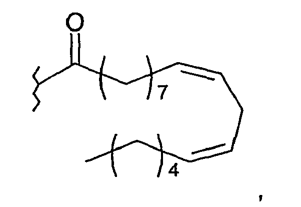 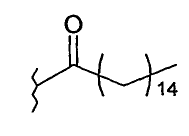 oder 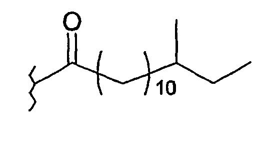 ist.

Das Lipopeptid-Ausgangsmaterial der Formel (I) ist fermentativ zugänglich. Beispiele für Lipopeptide der Formel (I) sind:
- Aculeacin (US 4212858)
- Desoxymulundocandin (EP 438813)
- Echinocandin-Derivate, z.B. Echinocandin B, C oder D (EP 1189933; FEBS Letters 1984, 173(1), 134-138)
- FR901379 (Biochim. Biophys. Acta 2002, 1587, 224-233)
- Mulundocandin (Bioorg. Med. Chem. Lett. 2004, 14, 1123-1128)
- Syringomycin (FEBS Letters 1999, 462, 151-154)

Bevorzugt kann in dem erfindungsgemäßen Verfahren Desoxymulundocandin, Echinocandin B oder Mulundocandin als Substrat eingesetzt werden.

Die Bereitstellung deacylierter Verbindungen der Formel (II) ermöglicht die Herstellung von semi-synthetischen Lipopeptiden (I') mit verbesserten pharmakologischen, pharmakokinetischen oder chemotherapeutischen Eigenschaften, wobei in einem weiteren Schritt (e) der Nukleus (II) mit einem Säurederivat re-acyliert wird, beispielsweise einer entsprechenden Säure in Gegenwart von Dimethylaminopyridin (DMAP) in Dimethylformamid (DMF) (J. Antibiot. 1999, 52(7), 674-676). Beispielsweise ist FK463 aus FR901379 mittels Deacylierung mit FR901379-Acylase über den entsprechenden Nukleus FR179642 und anschließende Acylierung mit einer Isoxazolyl-enthaltenden Benzoylseitenkette zugänglich (Biochim. Biophys. Acta 2002, 1587, 224-233).

Auch semi-synthetisch hergestellte Verbindungen der Formel (I) lassen sich in dem erfindungsgemäßen Verfahren zu der entsprechenden Verbindung der Formel (II) deacylieren.

Semi-synthetische Lipopeptide (I') entsprechen Verbindungen der Formel (I), unterscheiden sich aber in der Definition des Restes R₁₁. In semi-synthetischen Lipopeptiden (I') sind die Reste R₁-R₁₀ wie für die Verbindung (I) definiert, und die Gruppe R₁₁ ist definiert als eine Gruppe C(O)-Phenyl-(C₅-C₈)Heteroaryl-Phenyl, C(O)-Biphenyl oder C(O)-Terphenyl, wobei die Phenyl-, Biphenyl, Terphenyl- oder Heteroarylgruppen unsubstituiert sind oder substituiert sind mit einer oder zwei Gruppen ausgewählt aus der Gruppe (C₁-C₁₀)Alkyl oder O(C₁-C₁₀)Alkyl. In Verbindungen (I') können Phenylreste unsubstituiert sein oder einfach oder mehrfach, zum Beispiel einfach, zweifach oder dreifach, durch gleiche oder verschiedene Reste substituiert sein. In monosubstituierten Phenylresten kann sich der Substituent in der 2-Position, der 3-Position oder der 4-Position befinden. Zweifach substituiertes Phenyl kann in 2,3-Position, 2,4-Position, 2,5-Position, 2,6-Position, 3,4-Position oder 3,5-Position substituiert sein. In dreifach substituierten Phenylresten können sich die Substituenten in 2,3,4-Position, 2,3,5-Position, 2,4,5-Position, 2,4,6-Position, 2,3,6-Position oder 3,4,5-Position befinden. (C₅-C₈)Heteroarylreste sind aromatische Ringverbindungen mit insgesamt 5, 6, 7 oder 8 Atomen, in denen ein oder mehrere Ringatome Sauerstoffatome, Schwefelatome oder Stickstoffatome sind, z. B. 1, 2 oder 3 Stickstoffatome, 1 oder 2 Sauerstoffatome, 1 oder 2 Schwefelatome oder eine Kombination aus verschiedenen Heteroatomen, z.B. ein Stickstoff- und ein Sauerstoffatom. Die Heteroarylreste können über alle Positionen angebunden sein, zum Beispiel über die 1-Position, 2-Position, 3-Position, 4-Position, 5-Position, 6-Position, 7-Position oder 8-Position. Heteroarylreste können unsubstituiert sein oder einfach oder mehrfach durch gleiche oder verschiedene Reste substituiert sein. Heteroaryl bedeutet zum Beispiel Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl und Cinnolinyl. Als Heteroarylreste gilt insbesondere Isoxazolyl.

Verbindungen der Formel (I') sind beispielsweise beschrieben in der europäischen Patentanmeldung EP1189933.

Beispiele für semi-synthetische Lipopeptide der Formel (I') sind:
- A-1720132 (Antimicrob. Agents Chemother. 1998, 42, 389-393)
- A-192411.29 (Antimicrob. Agents Chemother. 2000, 44, 1242-1246)
- Caspofungin (MK-0991; Antimicrob. Agents Chemother. 1997, 41, 2326-2332)
- FK463 (Antimicrob. Agents Chemother. 2000, 44, 57-62)
- Pneumocandine, z.B. Pneumocandin A oder B (Tetrahedron Lett. 1992, 33(32), 4529-4532)

Ferner ist es mit dem erfindungsgemäßen Verfahren möglich, aus fermentativ zugänglichen Lipopeptiden (I) durch Deacylierung/Re-acylierung andere prinzipiell fermentativ zugängliche Lipopeptide herzustellen.

Ein besonders bevorzugtes Beispiel eines Lipopeptids (I) ist das Desoxymulundocandin (V) 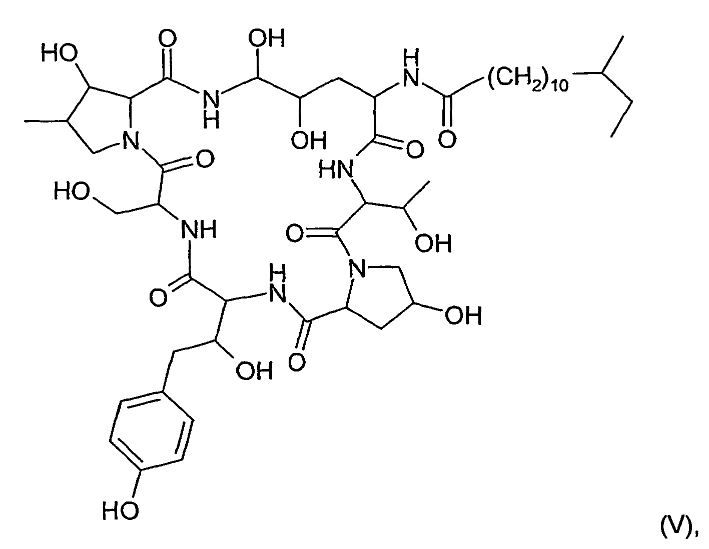 wobei in dem erfindungsgemäßen Verfahren durch Deacylierung ausgehend von Desoxymulundocandin (V) der Desoxymulundocandin-Nukleus (VI) hergestellt wird:

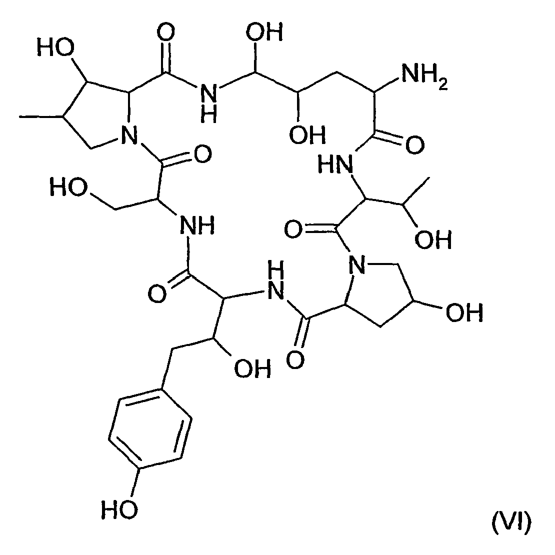

Lipopeptide der Formel (III) sind aus den europäischen Patentanmeldungen EP629636 und EP1068223 bekannt.

Das erfindungsgemäße Verfahren betrifft bevorzugt Verbindungen der Formel (III), in denen R₁₂, R₁₃, R₁₄, R₁₅, R₁₆, R₁₇, R₁₈, R₁₉, Rₓ und R_{y} gleich H sind.

Das erfindungsgemäße Verfahren betrifft ferner bevorzugt Verbindungen der Formel (III), in denen R₂₀ 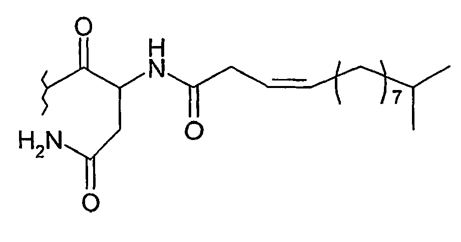 ist.

Ein besonders bevorzugtes Beispiel eines Lipopeptids (III) ist die Verbindung der Formel (VII) 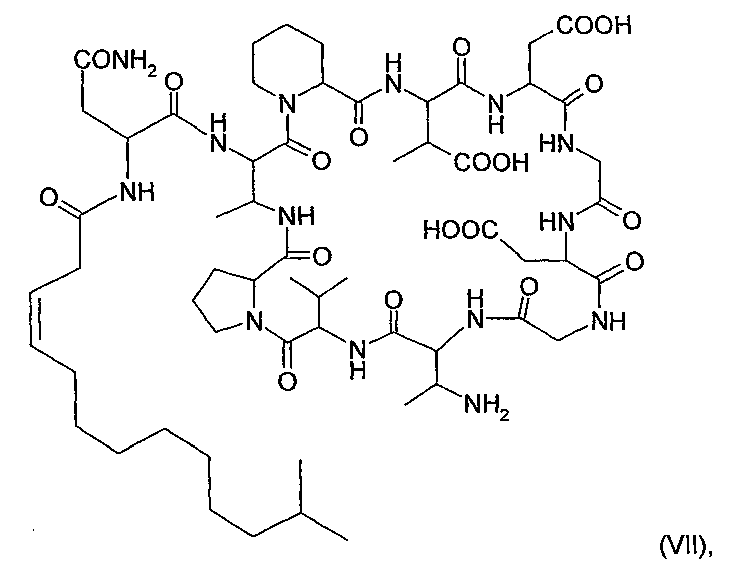 wobei durch Deacylierung der entsprechende Nukleus (VIII) hergestellt wird:

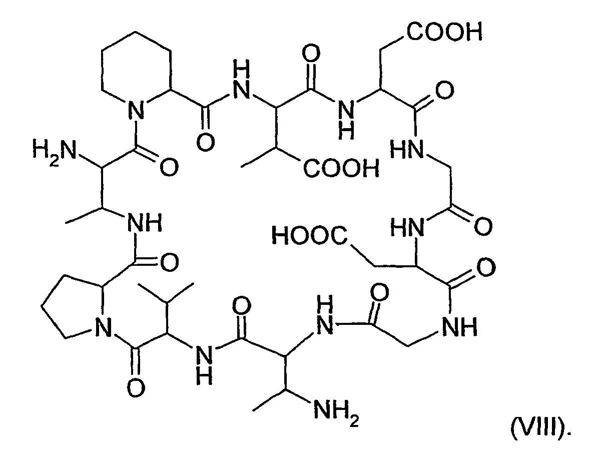

Ein Vorteil des erfindungsgemäßen Verfahrens ist, dass als Substrat der Deacylierung ein ungereinigtes, der Biomasse noch anhaftendes Lipopeptid (I) bzw. (III) in wässriger Suspension verwendet wird, die Aufreinigung des Ausgangsmaterials obsolet wird und so eine Produktion des Nukleus (II) bzw. (IV) in großtechnischem Maßstab möglich wird.

Zur Deacylierung werden natürliche Enzyme mit breitem Wirkungsspektrum in reiner oder partiell gereinigter Form eingesetzt. Beispiele für Deacylasen sind Echinocandin B (ECB) Deacylase, die durch Kultivierung einer *Actinoplanes* utahensis-Spezies erhalten wird (La Verne et al., J.Antibiotics 1989, 42, 382-388), oder Polymyxin-Deacylase (161-16081 Fatty Acylase, Pure; 164-16081 Fatty Acylase, Crude; Wako Pure Chemical Industries, Ltd.). Die ECB Deacylase aus *Actinoplanes utahensis* kann ebenso in *Streptomyces lividans* geklont und exprimiert werden. Die Herstellung eines rekombinanten Enzyms breiter Substratspezifität in einem transformierten *Streptomyces lividans*-Stamm ist bevorzugt, weil auf diese Weise signifikant höhere Enzymausbeuten zu erzielen sind. Die Deacylase kann in Schritt (c) in isolierter Form als Lösung oder als Feststoff zu der Biomasse der Lipopeptid-Fermentierung gegeben werden und wird vorzugsweise zuvor separat hergestellt und aufkonzentriert. Methoden zur Aufreinigung von Lipopeptid-Deacylase aus *Actinoplanes utahensis* sind literaturbekannt (siehe EP460882). Die Deacylierung wird nach Standardmethoden durchgeführt. Zum Beispiel wird die Deacylierung mit Polymyxin acylase nach Yasuda et al, Agric. Biol. Chem., 53,3245 (1989) und Kimura, Y., et al.,Agric. Biol. Chem., 53,497 (1989) durchgeführt.

### Beispiel 1: Herstellung des rekombinanten Deacylase-Produzenten

Aus dem Stamm *Actinoplanes utahensis* NRRL 12052 wurde ein DNS-Fragment isoliert, das für die Deacylase codiert. Das Fragment wurde kloniert (Plasmid pCS1) und umkloniert (Plasmid pCS2), und das Plasmid pCS2 in einen *S*. *lividans* Stamm transformiert. Von einer Einzelkolonie des rekombinanten S.lividans-Stammes wurden Sporensuspensionen hergestellt und bei -20°C in Ampullen eingelagert.

Klonierung der Deacylase: Die chromosomale DNS von *Actinoplanes utahensis* NRRL 12052 wurde isoliert, mit *Eco*RI und *Bgl*II verdaut und auf einem Agarosegel getrennt, DNS-Fragmente mit einer Länge von 7-9 kb wurden isoliert und in das Plasmid pUC19 kloniert (*Eco*RI/*Bam*HI Schnittstelle). Die Ligation wurde in E. coli transformiert und 250 resultierende Ampicillin-resistente Klone (50 µg/ml) wurden mittels PCR untersucht. Die Primer wurden auf Basis der publizierten DNS-Sequenz der Deacylase synthetisiert (siehe GenBank Nucleotide Sequence Database Accession Number D90543; J.Inokoshi et al, Gene 1992, 119, 29-35).

Das Plasmid pCS1 enthält das gesuchte 8 kb *Eco*RI/*Bgl*II Fragment aus *A. utahensis* NRRL 12052. Dies wurde auch durch Restriktionsverdau des Fragments und Vergleich mit den Literaturdaten bestätigt (siehe J.Inokoshi et al, Gene 1992, 119, 29-35). Das Plasmid pCS1 wurde mit *Eco*RI/*Hin*dIII verdaut und das 8 kb Fragment mit der Acylase in das Plasmid pWHM3 (*Eco*RI/*Hind*III-Schnittstelle; siehe J.Vara et al, J. Bacteriology 1989, 171, 5872-5881) kioniert und in *E. coli* transformiert. Die Transformanten wurden mit Ampicillin selektioniert (50µg/ml). Das resultierende Plasmid pCS2 enthält das 8 kb *Eco*RI/*Hin*dIII-Fragment mit der Deacylase.

Das Plasmid pCS2 wurde danach durch Polyethylenglykol (PEG)-Protoplastentransformation in den Stamm *Streptomyces lividans* TK64 JT46 transformiert, auf R2YE Medium (Kieser et al., Practical Streptomyces Genetics, The John Innes Foundation, 2000, page 408), ausplattiert und Klone mit Thiostrepton nach 24 Stunden selektioniert (20 µg/ml). Resultierende Thiostreptonresistente Transformanten wurden vereinzelt (in Medium 1) und ihre Produktivität in TSB-Medium in Schüttelkolben geprüft. Von einem positiven Klon wurden dann Sporensuspensionen hergestellt (Medium 1) und diese bei -20 °C in 20% Glycerol eingelagert. Allgemeine molekularbiologische Methoden (Ligation, Transformation, PCR, Restriktionsverdau, Agarosegele) sind bei Sambrook et al. (in "Molecular Cloning", Cold Spring Habor Laboratory Press, Second Edition, 1989, ISBN 0-87969-906-6) nachzulesen und Methoden zur Isolierung chromosomaler DNS aus Actinomyceten und Transformation von *Streptomyces lividans,* spezielle Methoden zur PCR von DNS aus Actinomyceten sowie Regeneration der Protoplasten sind bei Hopwood et al. (in "Practical Streptomyces Genetics", The John Innes Foundation, 2000, ISBN 0-7084-0623-8) zu finden.

Das im voranstehenden Abschnitt erwähnte Medium 1 enthält folgende Bestandteile:

| | |
|---|---|
| Malzextrakt | 10 g/L |
| Hefeextrakt | 4 g/L |
| Glucose | 4 g/L |
| Agar | 15 g/L |
| pH | 7,2 |

### Beispiel 2: Fermentative Herstellung der Deacylase

Zur Produktion des Enzyms wurde ein rekombinanter *Streptomyces lividans*-Stamm verwendet, wie er in Beispiel 1 beschrieben wurde. Ein solcher Stamm kann in einer Batch- oder Fed-Batch-Fermentation kultiviert werden. Die normale Prozesskette enthielt eine Vorkultur (TSB-Medium) im Schüttelkolben, welche drei Tage bei 28 °C und 220 rpm inkubiert wurde. Mit diesem Kolben kann ein Fermenter bis 2000 L direkt angeimpft werden (0,15 - 6 % v/v Inokulum). Alternativ dazu kann auch eine zweite Vorkultur im 10-50 L Maßstab in die Prozesskette eingefügt werden, um durch Erhöhung des Inokulums die Anwachsgeschwindigkeit in der Hauptstufe zu beschleunigen.

Fermentative Herstellung des Enzyms: Für die Reaktivierung der Sporen wurde ein Röhrchen einer Ampulle langsam aufgetaut. Mit 200µl der enthaltenen Suspension wurde ein Erlenmeyerkolben steril beimpft, der 500 ml des folgenden Medium enthielt [zusätzlich 10 µg/ ml Thiostrepton (50 mg/ml DMSO)]:

Medium der Vorkultur: TSB-Medium (Soybean-Casein-Digest Medium U.S.P.; ready prepared medium; OXOID LTD, England; product-no. CM129)

| Substanz | Konzentration [g/L] |
|---|---|
| Casein (digest) | 17 |
| Sojabohnenmehl (digest) | 3 |
| NaCl | 5 |
| K₂HPO₄ | 2,5 |
| Glucose | 2,5 |
| Wasser | ad 500ml |

| | |
|---|---|
| pH-Wert : | 7,3 ± 0,02, |
| Sterilisation : | 20 min, 121 °C, 1 bar, |
| Inkubation : | 3 Tage; 28 °C und 220 rpm (Hub: 2,5 cm). |

Am Ende der Vorkultur wurde diese den Animpfvorgaben entsprechend in einen Fermenter transferiert, der folgendes Medium enthält:

### Medium der Hauptkultur:

| Substanz | Konzentration [g/L] |
|---|---|
| (NH₄)₂SO₄ | 21 |
| K₂HPO₄ | 1 |
| Natriumglutamat | 5 |
| CaCl₂ | 1 |
| 50 x Mineralsalzlösung 1 | 20 ml |
| Desmophen 3600 | 1 ml |
| Glucose | 40 |
| Zitronensäure | 3,75 mg |

Die dem Hauptkulturmedium zugesetzte Mineralsalzlösung 1 besitzt folgende Zusammensetzung:

### Mineralsalzlösung 1:

| Substanz | Konzentration |
|---|---|
| | [g/L] |
| MgSO₄ * 7 H₂O | 28,9 |
| FeSO₄ * 7 H₂O | 0,5 |
| ZnSO₄ * 7 H₂O | 0,5 |
| MnSO₄ * 7 H₂O | 0,1 |
| CuSO₄ * 7 H₂O | 0,05 |
| CoCl₂ * 6 H₂O | 0,04 |

Die Sterilisation des Mediums kann mit Direktdampf oder im Autoklaven durchgeführt werden (bei 121-125 °C und 1,1-1,2 bar). Nach der Sterilisation betrug der pH-Wert etwa pH 6,5. Die Kohlenstoffquelle wurde separat und steril zugeführt, wobei der pH-Wert weiter auf etwa pH 6 abfiel. Nach Auffüllen des Mediums auf das gewünschte Volumen wurden während der Fermentation folgende Fermentationsbedingungen eingehalten:
Temperatur: 25-33 °C, vorzugsweise 28°C,
Druck: 0,5-1 bar, vorzugsweise 0,5 bar
Tip speed des Rührers: 1-2 m/s,
Begasung: 0,25-1,5 wm, vorzugsweise 0,5 vvm,
pO2: >10 % (kontrolliert)
pH-Regelung bei 6,5-7,2 mit Phosphorsäure und/oder Natronlauge,
vorzugsweise pH 7,0 (kontrolliert).

Optional kann ein Antischaummittel eingesetzt werden, beispielsweise ein verzweigter, Hydroxylgruppen-haltiger Polyester, vorzugsweise Desmophen® (Bayer Material Science, Leverkusen, Germany).

Nach etwa 100 Stunden war die Kohlenstoffquelle verbraucht und es fanden sich zwischen 50-150 U/ L in der Fermentationslösung.

Nach Einstellen dieser Parameter und Animpfen mit dem gewünschten Inokulum-Volumen wurde das Produktivitätsmaximum nach 72-120 Stunden erreicht. Die Expression des Enzyms erfolgte wachstumsgekoppelt und musste nicht induziert werden. Zur Überwachung der Expression bzw. der Produktion des Enzyms wurde für die Bestimmung der Enzymaktivität ein offline Schnelltest (siehe Beispiel 3) eingesetzt. Die Fermentation wurde zum Zeitpunkt maximaler Produktivität beendet. Durch Anwendung eines Fed-Batch-Verfahrens konnte die Produktivität und die Raum-Zeit-Ausbeute beträchtlich gesteigert werden. Hierzu wurde bevorzugt eine Glucose-Lösung (1-5 g/l*h) im Fed-Batch unter gleichzeitiger Erhöhung des Leistungseintrags über die Rührergeschwindigkeit (pO₂-Regelung > 10 %) verwendet.

### Beispiel 3: Aktivitätsbestimmung der Deacylase

Zur Kontrolle der Enzymaktivität während der fermentativen Herstellung der Deacylase, während der Isolierung des Enzyms oder während der enzymatischen Seitenkettenabspaltung aus Lipopeptiden wie Desoxymulundocandin dient folgender enzymatischer Schnelltest:

Zu 500 µL einer auf 60 °C vorgeheizten Lösung von 2,5 g/L Desoxymulundocandin (V), 0,5 %v/v Brij 35 in 200mM Natriumphosphatpuffer (pH 5,5) werden 20 µL einer die Deacylase-enthaltenden Probe gegeben. Die Lösung wird unter Schütteln 10 Minuten bei 60 °C inkubiert. Die Deacylierung wird anschließend durch Zugabe von 480 µL 0,85 %v/v Phosphorsäure gestoppt.

Nach Zentrifugation und Abtrennung von unlöslichen Bestandteilen wird die Menge des gebildeten Desoxymulundocandin-Nukleus (VI) in einer begleitenden HPLC-Analytik bestimmt. Hierzu werden 5 µL der Lösung auf eine Merck-Purospher-Star-RP-Säule (4*55mm) injiziert und mit einem dreiminütigen, linearen Gradienten von 4→20 %v/v Acetonitril, angesäuert mit 0,1 %v/v Phosphorsäure, bei einem Fluss von 2,5 ml/min eluiert. Die Detektion erfolgt bei λ = 220 nm. Der Nukleus besitzt eine Retentionszeit von 1,4 Minuten. Die Quantifizierung erfolgt mittels eines externen Standards.

Eine Einheit (Unit) (U) an Deacylase-Aktivität wird definiert als die Menge an Enzym, die unter den beschriebenen Analysenbedingungen benötigt wird, um in einer Minute 1 µmol an Desoxymulundocandin-Nukleus zu produzieren.

Hochkonzenztrierte Enzymproben, wie sie z.B. während der Isolierung der Deacylase anfallen, werden mit 200 mM Natriumphosphatpuffer (pH 5,5) auf eine im Enzymtest korrekt quantifizierbare Konzentration von 5-200 U/mL verdünnt. Bei Enzymproben, die Desoxymulundocandin oder den Desoxymulundocandin-Nukleus enthalten, wird ein Analogsubstrat, zum Beispiel die Verbindung (VII) im Schnelltest verwendet. Der resultierende Nukleus (VIII) wurde bei einer Retentionszeit von 2,4 Minuten detektiert.

### Beispiel 4: Isolierung der Deacylase

Zur Isolierung des Enzyms wurde der Überstand von Kulturlösungen, die wie voranstehend beschrieben hergestellt wurden, mittels an sich bekannter Methoden, vorzugsweise Zentrifugation abgetrennt und danach aufkonzentriert, beispielsweise mittels Ultrafiltration. Beispielsweise werden nach beendeter Fermentation Kulturlösungen (ca. 2000 L) des rekombinanten *Streptomyces lividans*-Stammes gegebenenfalls mit geeigneten Desinfektionsmitteln abgetötet und der das Enzym enthaltende Überstand mit einem Separator (z.B. Typ Westfalia SC 35) bei einem Durchsatz von 1000-1300 l/h abgetrennt. Die abgetrennte Biomasse wurde verworfen. Die Qualität der Fest-Flüssig-Trennung kann durch Messung der optischen Dichte bei λ = 540 nm im Klarlauf getestet werden. Für die Aufkonzentrierung des geklärten Überstandes ist bevorzugt ein Konzentrierungsfaktor von etwa 10-20 im Vergleich zu seinem ursprünglichen Volumen unter Verwendung von Polyethersulfon-Membranen mit einer nominalen Ausschlussgrenze (cut-off) von 10-50 kDa, vorzugsweise um 20 kDa. Die Zirkulationsflussrate betrug 4500 l/h bei einem Trans-Membran-Druck von 2,5-3,5 bar; der Permeatfluss ging während der Filtration von anfänglich 20-30 l/hm² auf 4-10 l/hm² zurück. Das Permeat wurde verworfen.

Die wässrigen Enzym-Konzentrate (Retentate) konnten in dieser Form kühl gelagert und danach direkt zur Deacylierung von Lipopeptiden eingesetzt werden.

Alternativ dazu gelang es auch, das Enzym durch Zusatz von 2-Propanol mit einer Endkonzentration von 40-60 %v/v, vorzugsweise 50-55 %v/v, zu fällen. Die Fällung kann im Temperaturbereich von 0-25 °C, vorzugsweise im Bereich von 4-10 °C, besonders bevorzugt 6°C vorgenommen werden. Die Deacylase lässt sich anstelle von 2-Propanol auch mit Aceton fällen, 1-Propanol ist jedoch als Fällungsmittel weniger gut geeignet. Der Überstand wurde dekantiert und danach das Enzym aus der verbleibenden Suspension nach beendeter Fällung abzentrifugiert (z.B. mit einer CEPA-Röhrenzentrifuge) und in feuchter Form bis zur weiteren Verwendung kühl gelagert.

Das feuchte, in Pellets anfallende Enzym wurde in einem Phosphatpuffer geeigneter Konzentration gelöst. Unlösliche Bestandteile werden abfiltriert. Das klare Filtrat wurde nach Aktivitätsbestimmung direkt zur Deacylierung des Desoxymulundocandins eingesetzt.

Das Präzipitat kann ferner nach vorherigem Zusatz von Additiven wie Salzen (beispielsweise Ammoniumsulfat), Zuckern (z.B. Glucose) oder Zuckeralkoholen wie Sorbitol oder Mannitol in lyophilisierter Form über einen längeren Zeitraum stabil aufbewahrt werden. Die Verwendung eines Enzympräzipitats ist bevorzugt, weil auf diese Weise Medienkomponenten der *Streptomyces lividans*-Fermentation vor der Seitenkettenabspaltung eliminiert werden können.

### Beispiel 5: Fermentative Herstellung von Lipopeptiden

Zur Herstellung zyklischer Peptide der Formel (II), auch Nukleus genannt, wurden zuvor die entsprechenden, als Substrat zu verwendeten Lipopeptide der Formel (I) (exozyklische N-Acyl-Derivate des Nukleus) durch Kultivierung der betreffenden Mikroorganismen fermentativ gewonnen. Zum Beispiel erhält man das wasserunlösliche, der Biomasse anhaftende Desoxymulundocandin (V) durch Kultivierung von *Aspergillus sydowii*, wie dies in der Europäischen Patentanmeldung 0 438 813 A1 beschrieben ist und nachfolgend nochmals vorgestellt wird:

Zur Produktion von Desoxymulundocandin wurde ein Produzent von *Aspergillus sydowii*, vorzugsweise ein durch klassische Stammverbesserung ausgewählter Produzent, in einem Batch Fermentationsprozess vermehrt. Desoxymulundocandin ist ein klassischer Sekundärmetabolit, der erst nach ca. drei Tagen Fermentationsdauer gebildet wird:

Der Desoxymulundocandin produzierende *Aspergillus sydowii* Stamm wurde in einem Erlenmeyerkolben als Vorkultur angezogen. Die Kolben wurden mit 1 ml einer Sporensuspension direkt aus der Ampulle angeimpft. Folgende Parameter wurden eingehalten: T = 28 °C; 240 rpm, 48-72 Stunden.

Als Vorkulturmedium wurde eingesetzt:

| Substanz | Menge [g/L] |
|---|---|
| Corn Steep Pulver | 0,5 |
| CaCO₃ | 2 |
| Hefeextrakt | 2 |
| Glucose | 5 |
| NaCl | 5 |
| Sojamehl | 15 |
| Pharmamedia | 15 |
| Desmophen | 3 ml/L |

Der pH Wert lag nach der Sterilisation des Mediums bei pH 6.9 und betrug am Ende der Kultur pH 7-7,5. Der PMV-Wert (percent mycelial volume) lag zwischen 15-20 %. Für einen industriellen Prozess wurde eine zweite Vorkultur fermentiert, die unter den gleichen Bedingungen und mit dem gleichen Medium durchgeführt wurde.

Der Hauptfermenter wurde mit 1-6% Inokulum aus einer Schüttelkultur oder einem Vorfermenter beimpft und enthielt folgendes Medium:

### Hauptkulturmedium (MF3)

| Substanz | Anteil [%] |
|---|---|
| Hefeextrakt | 0,930 |
| Sojamehl | 1,430 |
| Pharmamedia | 1,475 |
| lösliche Stärke | 4,300 |
| CaCO₃ | 0,400 |
| MgSO₄*7H₂O | 0,070 |
| Desmophen | 0,200 |
| Glucose | 1,000 |
| Standardspurenelementlösung | 1 ml/L |

### Fermentationsparameter:

Animpfdichte: 0,5-6%, bevorzugt 1-2 %,
Temperatur: 25-33 °C, bevorzugt 27-30 °C,
Begasung: 0-1,5 vvm, bevorzugt 0,5-0,8 vvm,
Tip speed des Rührers: 1,4 m/s (abhängig vom verwendeten Rührer)

Die Fermentation wurde bei T= 25-35 °C, bevorzugt bei 28-32°C durchgeführt. Es wurde ein leichter Überdruck von 0,2-1bar angewandt und kontinuierlich eine Konzentration an gelöstem Sauerstoff >30% geregelt. Hierzu verwendete man bevorzugt eine Kaskade aus steigender Rührergeschwindigkeit (startend mit 95 rpm, Tipp speed 1,2 m/s) und steigender Begasungsrate (startend mit 0.2 N/m³). Der pH-Wert mußte während der Fermentation nicht geregelt werden. Zur Verbesserung der Prozessrobustheit ist es aber dienlich. Der pH-Wert lag im Bereich von 5,5-7; bevorzugt 6,2-6,5. Ab der 24-30. Stunde wurde der pH-Wert mit Schwefelsäure auf pH 6.5 geregelt. Zur Vermeidung von Schaumbildung können unterschiedliche Entschäumer verwendet werden, bevorzugt sind Desmophen 3600 oder Hodag AFM-5. Zur Produktion von Desoxymulundocandin ist eine bevorzugte Morphologie über den gesamten Fermentationsprozess anzustreben. Dieses kleine Pellet-förmige Wachstum wird durch die Wahl des Rührers beeinflusst; ein Scheibenrührer ist für Pellet-artiges Wachstum besonders geeignet.

Die Gesamtfermentationsdauer kann mehr als 240 Stunden betragen, wobei die Produktbildungsrate über einen langen Zeitraum recht konstant verläuft und das Fermentationsende über eine begleitende offline-Analytik festgelegt wird. Die Produktkonzentration am Ende einer Fermentation lag im Bereich von 800-1200 mg/l an Desoxymulundocandin.

Vor dem Ernten der Biomasse wird der pH-Wert auf einen pH-Wert von 6,5 bis 5,5, vorzugsweise pH 6,0 eingestellt, dem Stabilitätsoptimum des Desoxymulundocandins.

### Beispiel 6.1: Deacylierung von Lipopeptiden zum entsprechenden Nukleus (1)

Nach beendeter Fermentation wurde die Kulturbrühe in Kulturüberstand und Biomasse getrennt. Hierzu können verschiedene, gängige Filtrations- und Separationstechniken genutzt werden. Vorzugsweise wird eine Filterpresse verwendet. Die Biomasse wurde auf der Filterpresse gegebenenfalls einmal mit Wasser gewaschen und danach für die Deacylierung in einen Reaktionsbehälter übergeführt. Filtrat und Waschwasser wurden verworfen.

Anschließend wurde die Biomasse zusammen mit dem anhaftenden Lipopeptid in Wasser re-suspendiert, vorzugsweise in der 1-2fachen Menge an Wasser. Beispielsweise wurde nach beendeter Desoxymulundocandin-Fermentation 30-50 kg an feuchter Biomasse in 100-150 Liter Wasser re-suspendiert, wobei die Ausgangskonzentration an Desoxymulundocandin zur Deacylierung zwischen 0,5-1,5 g/L lag. Die Suspension wurde solange gerührt, bis ein homogener Brei vorlag.

Die Suspension wurde auf den gewünschten pH-Wert eingestellt. Zur Seitenkettenabspaltung wurde dann das Reaktionsgemisch mit einer Lösung der Deacylase versetzt, wobei 20-50 Units an Enzym pro Gramm Lipopeptid, vorzugsweise 25-40 U/g, zur Anwendung kamen, entsprechend 25-150 U/L, bevorzugt 25-80 U/L.

Der Deacylierungsprozess selbst wurde - nach Auflösen des Enzyms in einem Phosphatpuffer - bei Temperaturen von 20-80 °C durchgeführt; bevorzugt war ein Temperaturbereich von 20-40 °C, besonders bevorzugt 30-35°C. Höhere Temperaturen können die Nebenproduktbildung wie Ringöffnung und/oder Dehydratisierung begünstigen. Die Rührergeschwindigkeit betrug in einem 200L-Fermenter 100-250 rpm, bevorzugt 120-180 rpm.

Die Deacylase kann im pH-Bereich von pH 4 bis 9 eingesetzt werden; bevorzugt ist ein Bereich von pH 4,6 bis pH 7,8 mit einem Wirkungsoptimum des Enzyms bei pH 5,2-6,2. Höhere pH-Werte sind zu vermeiden, da auch in diesem Fall die Bildung ringoffener oder dehydratisierter Nebenprodukte der Formeln (IX) und (X) verstärkt eintritt:

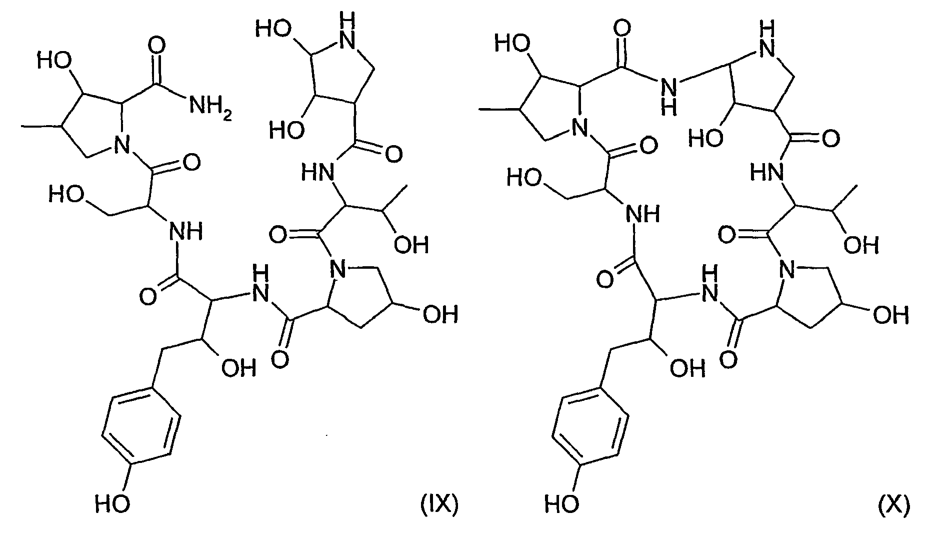

Die Reaktionszeit der Deacylierungsreaktion variiert und hängt stark vom gewählten pH-Wert und der gewählten Temperatur ab. Das Ende der enzymatischen Abspaltung der Seitenkettensäure wird mittels begleitender analytischer HPLC durch Messung der Nukleus-Bildung im Reaktionsüberstand bestimmt. Beispielsweise war bei einer Deacylierung von Desoxymulundocandin im 200L-Maßstab die Deacylierung nach 20-30 Stunden komplett abgelaufen.

### Beispiel 6.2: Deacylierung von Lipopeptiden zum entsprechenden Nukleus (2)

Die Deacylierung von Deoxymulundocandin wurde entsprechend Beispiel 6.1 durchgeführt. Dabei wurde das Deoxymulundocandin-Mycel abgepresst und mit Wasser gewaschen. Dann erfolgte die Re-suspension im gleichen Volumen Wasser und die Deacylierung zum Nucleus bei pH 5,5 und 30°C:
Kulturfiltrat: 160 Liter
Kulturfiltrat-Reinheit: 72,3 area% (HPLC)
Beladung: 3,8 g/L
Hauptfraktion: 55 Liter
Hauptfraktion-Reinheit: 95,1 area% (HPLC)
Lyophilisat-Nucleus: 34,1 g (Ausbeute 30%)
Lyophilisat-Reinheit: 88,0 area% (HPLC) - 78,4 %w/w (gegen internen Standard).

### Beispiel 7: Isolierung und Reinigung des Nukleus

Nach beendeter Deacylierung wurde die *Aspergillus sydowii*-Biomasse abzentrifugiert oder abfiltriert, wobei gegebenenfalls ein Filterhilfsmittels zugesetzt wurde. Die abgetrennte Biomasse wurde verworfen. Das klare Filtrat, das den wasserlöslichen Nukleus enthält, wurde anschließend säulenchromatographisch gereinigt. Als stationäre Phase diente ein hydrophobes Polymer wie Polystyren-Vinylbenzen-Copolymerisate, Polyacrylate oder Polymethacrylate. Alternativ dazu kann der Nukleus auch an einem Kationenaustauscher chromatographisch gereinigt werden. Bevorzugt ist eine säulenchromatographische Reinigung an einem Styren-Vinylbenzen-Copolymer als stationäre Phase.

Als Elutionsmittel (mobile Phase) diente Wasser, dem zur Erhöhung der Selektivität organische Säuren und Alkohole als Cosolventien zugesetzt wurden. Bevorzugt als mobile Phase ist Wasser, das geringe Mengen an Essigsäure und 1- bzw. 2-Propanol enthält. Der Säurezusatz dient zur gezielten Abreicherung vorhandener Verunreinigungen wie ringoffenen oder dehydratisierten Verbindungen.

Das Eluat wurde fraktioniert aufgefangen und die den Nukleus enthaltenen Fraktionen - nach erfolgter Quantifizierung mittels begleitender analytischer HPLC - vereinigt und durch Nanofiltration konzentriert. Für die Nanofiltration wurden Membranen eingesetzt, die 50-70 % Natriumchlorid, vorzugsweise 50 % NaCl, zurückhalten können.

Das erhaltene Nukleus-Konzentrat (Retentat) wurde anschließend gefriergetrocknet oder sprühgetrocknet. Der resultierende feste Nukleus konnte direkt und ohne zusätzliche Reinigung als Ausgangsmaterial für die Re-Acylierung mit aktivierten aromatischen Seitenkettensäuren eingesetzt werden.

### Isolierung und Reinigung des Desoxymulundocandin-Nukleus im großtechnischen Maßstab:

Nach beendeter enzymatischer Seitenkettenabspaltung wird ein 1000 Liter-Deacylierungsansatz über eine Filterpresse filtriert. Die abgetrennte Biomasse wird auf der Filterpresse mit Wasser gewaschen und danach verworfen. Filtrat und Waschwasser werden vereinigt und nochmals über eine Schicht (Seitz Typ K-200) klarfiltriert. Die filtrierte Lösung sollte einen pH-Wert von 6-6,5 besitzen. Gegebenenfalls ist der pH-Wert mit 2 M-Essigsäure oder 2 M-NatriumhydroxidLösung nachzustellen.

750 Liter der klaren Lösung, die zwischen 500-700 mg/L an Desoxymulundocandin-Nukleus enthalten kann, werden mit einem linearen Fluss von 100-200 cm/h auf eine Chromatographiesäule aufgetragen, die mit 25 Liter Amberchrom®-CG161m als stationäre Phase gefüllt ist. Die Betthöhe der stationären Phase beträgt 26 cm, der innere Säulendurchmesser 35 cm. Die Leitfähigkeit der Lösung ist unkritisch.

Während des gesamten Chromatographieverlaufs werden die Absorption bei λ= 280 nm, der pH-Wert und die Leitfähigkeit kontinuierlich gemessen und aufgezeichnet. Der Säulendurchlauf wird in einer Fraktion aufgefangen und nach HPLC-Testung verworfen.

Nach beendeter Beladung wird die stationäre Phase solange mit gereinigtem Wasser unter einem linearen Fluß von 100-150 cm/h gewaschen, bis die im Säulenauslauf kontinuierlich gemessene Absorption nahezu die Basislinie erreicht hat. Die Waschlösung wird in einer einzigen Fraktion gesammelt und nach HPLC-Testung verworfen.

Nach beendetem Waschen wird der Desoxymulundocandin-Nukleus unter einem linearen Fluss von 100-150 cm/h mit 10 Bettvolumina isokratisch eluiert. Zur Desorption wird Wasser, dem zur Erhöhung der Selektivität 0,1 %v/v Essigsäure und 2 %v/v 2-Propanol (oder 1-Propanol) zugesetzt werden, verwendet. Das Eluat wird in Fraktionen zu 5-25 Liter aufgefangen, wobei die Fraktionsgröße über die gemessene und kontinuierlich aufgezeichnete Absorption gesteuert wird.

Allen Fraktionen werden Aliquots entnommen und darin mittels begleitender HPLC-Analytik die Reinheit und der Gehalt an Desoxymulundocandin-Nukleus bestimmt. Nukleus-enthaltende Fraktionen mit einer Reinheit > 90 Area% werden vereinigt. Anschließend wird das Gesamteluat (50-100 Liter) mittels Nanofiltration um den Faktor 2-5 aufkonzentriert unter Verwendung einer Membran, deren Natriumchlorid-Rückhalt 50% beträgt.

Das erhaltene Nukleus-Retentat wird danach sterilfiltriert und dann gefriergetrocknet oder sprühgetrocknet. Randfraktionen der säulenchromatographischen Reinigung des Desoxymulundocandin-Nukleus können recyclisiert werden.

Nach vollständiger Trocknung wird der erhaltene, feste Desoxymulundocandin-Nukleus nach HPLC-Analyse in geeignete Kunststoffbehälter abgefüllt. Die Gebinde werden bis zur weiteren Verwendung bei -25 °C aufbewahrt.

## Patentansprüche

1. Verfahren zur enzymatischen Abspaltung der N-Acyl-Seitenkette aus Lipopeptiden unter Bildung des entsprechenden Nukleus, wobei
(a) das Lipopeptid fermentativ hergestellt wird, wobei das Lipopeptid an der Biomasse zellgebunden vorliegt, und die Biomasse mit dem anhaftenden Lipopeptid abgetrennt wird,
(b) die Biomasse mit dem anhaftenden Lipopeptid aus Schritt (a) in einem wässrigem System re-suspendiert wird,
(c) eine geeignete Deacylase in gelöster oder fester Form zu der Suspension der Biomasse aus Schritt (b) gegeben wird, und sich der entsprechende Nukleus bildet, und
(d) der Nukleus optional isoliert und gereinigt wird,
**dadurch gekennzeichnet, dass** das in Schritt (a) fermentativ gewonnene Lipopeptid nach Fermentationsende als zellgebundene Biomasse ohne weitere Reinigung in Schritt (c) direkt mit einer Deacylase zur Reaktion gebracht wird, wodurch die über eine Amidbindung verknüpfte N-Acylkette abgespalten wird.

2. Verfahren gemäß Anspruch 1, wobei das Lipopeptid die Formel (I) hat 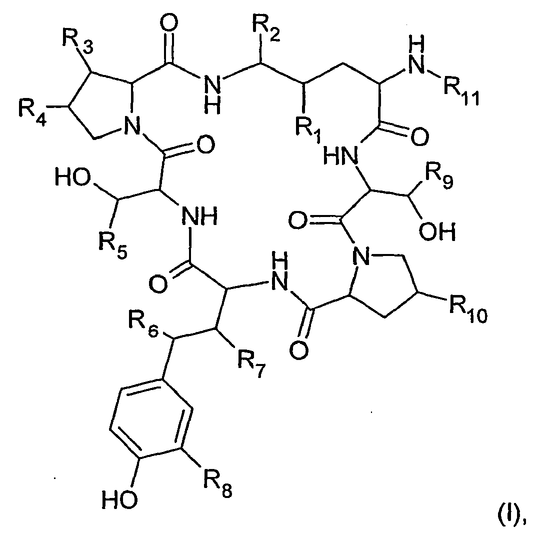 und wobei
R₁ = H, OH oder NRₓR_{y} ist, wobei
Rₓ und R_{y} unabhängig voneinander H oder (C₁-C₆)Alkyl sind,
R₂ = H, OH, NH(CH₂)₂NH₂,
R₃ = H, OH,
R₄ = H, Me, NH₂, -NH-C(=NH)NH₂,
R₅ = H, Me, CH₂-C(=O)NH₂, CH₂CH₂NH₂,
R₆ = H, OH,
R₇ = H, OH,
R₈ = H, OSO₃H, OSO₃Na, NH-C(=O)CH₂NH₂,
R₉ = Me, und
R₁₀ = H, OH,
R₁₁ = eine Gruppe C(O)-(C₆-C₂₄)Alkyl, eine Gruppe C(O)-(C₆-C₂₄)Alkenyl, eine Gruppe C(O)-(C₆-C₂₄)Alkadienyl, oder eine Gruppe C(O)-(C₆-C₂₄)Alkatrienyl,
unter Bildung des entsprechenden Nukleus der Formel (II) 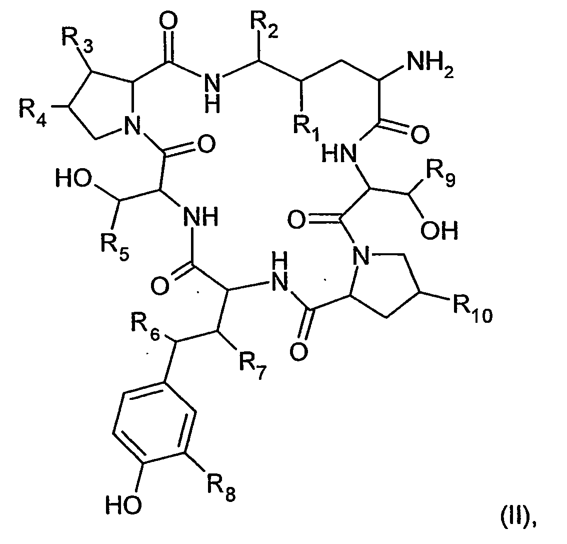 wobei R₁-R₁₀ die für Formel (I) genannte Bedeutung haben, und wobei sofern vorhanden Alkyl-, Alkenyl-, Alkadienyl- und Alkatrienylgruppen in Verbindungen der Formel (I) und (II) verzweigt oder geradketttig sein können.

3. Verfahren gemäß Anspruch 2, wobei in einem weiteren Schritt
(e) der Nukleus (II) mit einem Säurederivat zu einem semi-synthetischen Lipopeptid der Formel (I') umgesetzt wird, in dem R₁-R₁₀ die für Formel (I) genannte Bedeutung haben und die Gruppe R₁₁ definiert ist als eine Gruppe C(O)-Phenyl-(C₅-C₈)Heteroaryl-Phenyl, C(O)-Biphenyl oder C(O)-Terphenyl.

4. Verfahren gemäß einem der Ansprüche 2 oder 3, wobei der (C₆-C₂₄)Alkyl in Rest R₁₁ der Verbindung (I) n-Hexyl, n-Octyl, n-Decanyl, n-Undecanyl, n-Dodecanyl, n-Tridecanyl, n-Tetradecanyl, n-Pentadecanyl, n-Hexadecanyl, n-Heptadecanyl, n-Octadecanyl, n-Nonadecanyl, n-Eicosanyl, n-Dicosanyl, 9,11-Dimethyl-tridecanyl oder 11-Methyl-tridecanyl bedeutet.

5. Verfahren gemäß einem der Ansprüche 2 bis 4, wobei in Verbindung (I) R₁₁ 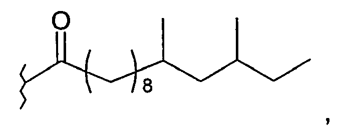 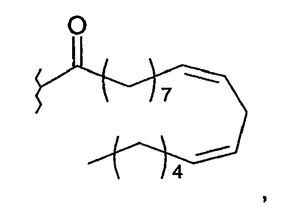 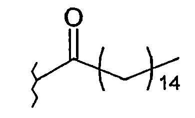 oder 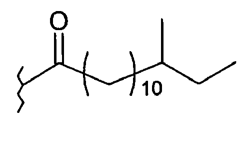 ist.

6. Verfahren gemäß einem der Ansprüche 2 bis 5, wobei in Schritt (a) die Verbindung Desoxymulundocandin (V) 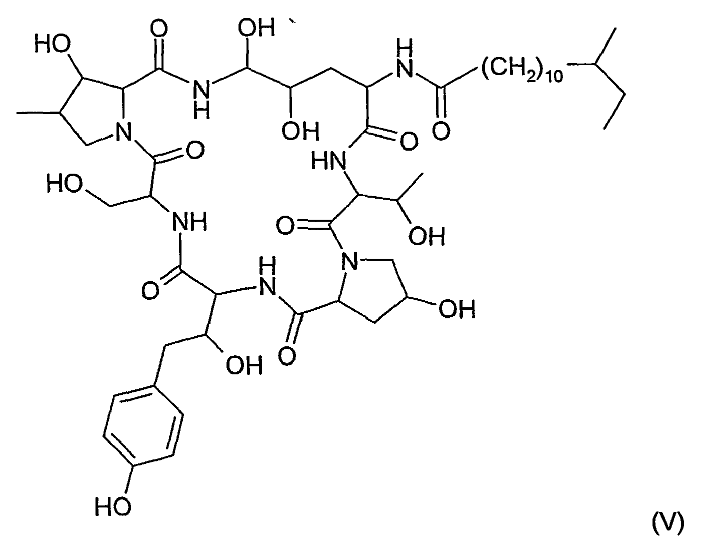 hergestellt wird,
und sich in Schritt (c) der Desoxymulundocandin-Nukleus (VI) 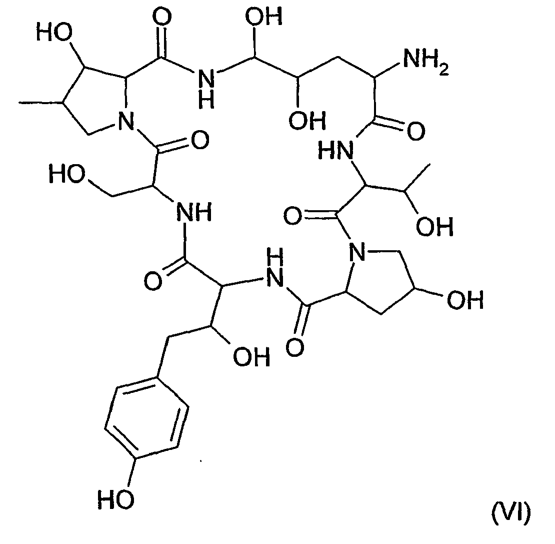 bildet.

7. Verfahren gemäß Anspruch 1, wobei das Lipopeptid die Formel (III) 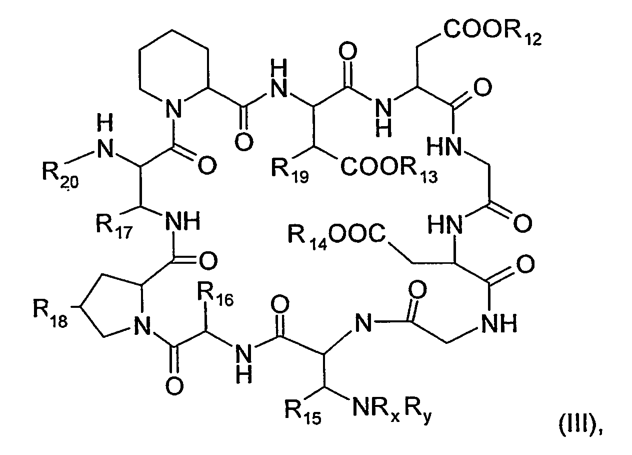 hat, wobei
R₁₂, R₁₃, R₁₄, R₁₅, R₁₆, R₁₇, R₁₈, R₁₉, Rₓ und R_{y} unabhängig voneinander H oder (C₁-C₆)Alkyl sind, und
R₂₀ = eine Gruppe C(O)-CH(CH₂COR₂₁)-NH-CO-(C₆-C₂₄)Alkyl,
eine Gruppe C(O)-CH(CH₂COR₂₁)-NH-CO-(C₆-C₂₄)Alkenyl, eine Gruppe C(O)-CH(CH₂COR₂₁)-NH-CO-(C₆-C₂₄)Alkadienyl, oder eine Gruppe C(O)-CH(CH₂COR₂₁)-NH-CO-(C₆-C₂₄)Alkatrienyl, wobei R₂₁ OH oder NH₂ ist,
unter Bildung des entsprechenden Nukleus der Formel (IV) 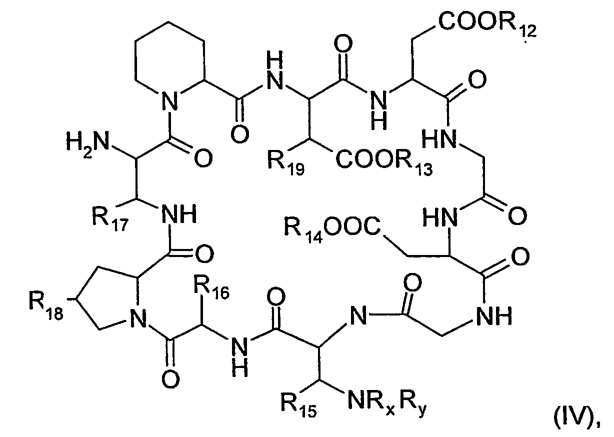 wobei R₁₂, R₁₃, R₁₄, R₁₅, R₁₆, R₁₇, R₁₈, R₁₉, Rₓ und R_{y} die für Formel (III) genannte Bedeutung haben, und wobei Alkyl-, Alkenyl-, Alkadienyl- und Alkatrienylgruppen sofern vorhanden in Verbindungen der Formel (III) und (IV) verzweigt oder geradketttig sein können.

8. Verfahren gemäß Anspruch 7, wobei R₁₂, R₁₃, R₁₄, R₁₅, R₁₆, R₁₇, R₁₈, R₁₉, Rₓ und R_{y} gleich H sind.

9. Verfahren nach einem der Ansprüche 7 oder 8, wobei R₂₀ 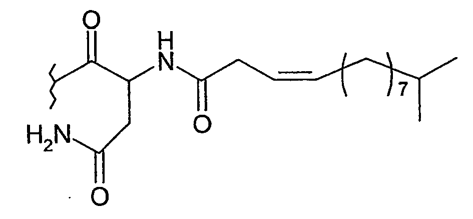 ist.

10. Verfahren nach einem der Ansprüche 7 bis 9, wobei in Schritt (a) die Verbindung (VII) 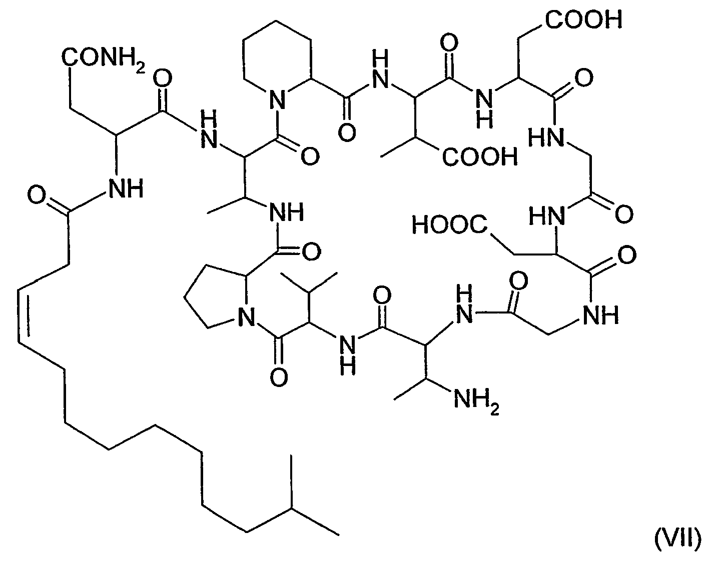 hergestellt wird,
und sich in Schritt (c) die Verbindung (VIII) 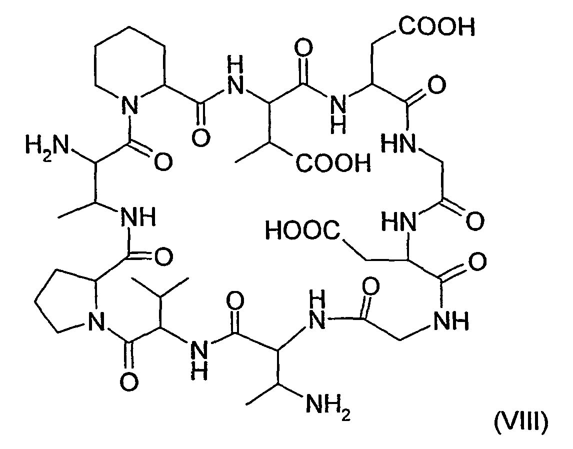 bildet.

11. Verfahren gemäß einem der Ansprüche 1 bis 10, wobei die Deacylase durch Kultivierung von *Actinoplanes utahensis* erhalten wurde.

12. Verfahren gemäß einem der Ansprüche 1 bis 10, wobei die Deacylase rekombinant durch Kultivierung von transformierten *Streptomyces lividans* erhalten wird.

## Claims

1. A method for the enzymatic cleavage of the N-acyl side chain from lipopeptides to form the corresponding nucleus, where
(a) the lipopeptide is prepared by fermentation, the lipopeptide being bound to the cells of the biomass, and the biomass is removed with the adhering lipopeptide,
(b) the biomass with the adhering lipopeptide from step (a) is resuspended in an aqueous system,
(c) a suitable deacylase is added in dissolved or solid form to the suspension of the biomass from step (b), and the corresponding nucleus is formed, and
(d) the nucleus is optionally isolated and purified,
wherein the lipopeptide obtained by fermentation in step (a) is reacted after the end of the fermentation as cell-bound biomass without further purification in step (C) directly with a deacylase, whereby the N-acyl chain linked via an amide linkage is cleaved.

2. The method as claimed in claim 1, where the lipopeptide has the formula (I) 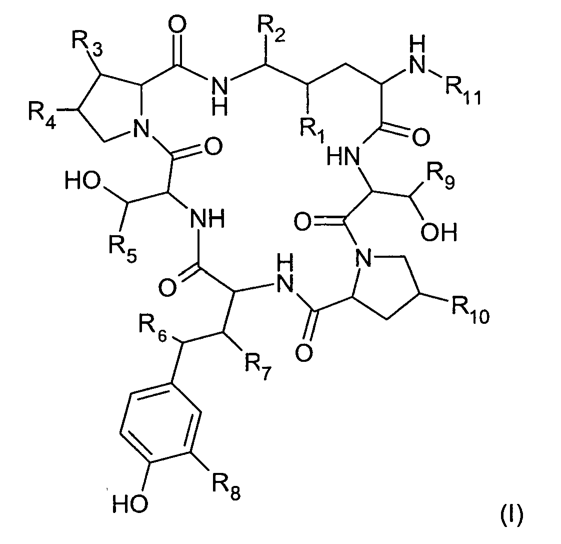 and where
R₁ is H, OH or NRₓR_{y}, where
Rₓ and R_{y} are independently of one another H or (C₁-C₆)alkyl,
R₂ is H, OH, NH(CH₂)₂NH₂,
R₃ is H, OH,
R₄ is H, Me, NH₂, -NH-C(=NH)NH₂,
R₅ is H, Me, CH₂-C(=O)NH₂, CH₂CH₂NH₂,
R₆ is H, OH,
R₇ is H, OH,
R₈ is H, OSO₃H, OSO₃Na, NH-C(=O)CH₂NH₂,
R₉ is Me, and
R₁₀ is H, OH,
R₁₁ is a C(O)-(C₆-C₂₄)alkyl group, a C(O)-(C₆-C₂₄)alkenyl group, a C(O)-(C₆-C₂₄)alkadienyl group, or a C(O)-(C₆-C₂₄)alkatrienyl group,
to form the corresponding nucleus of the formula (II) 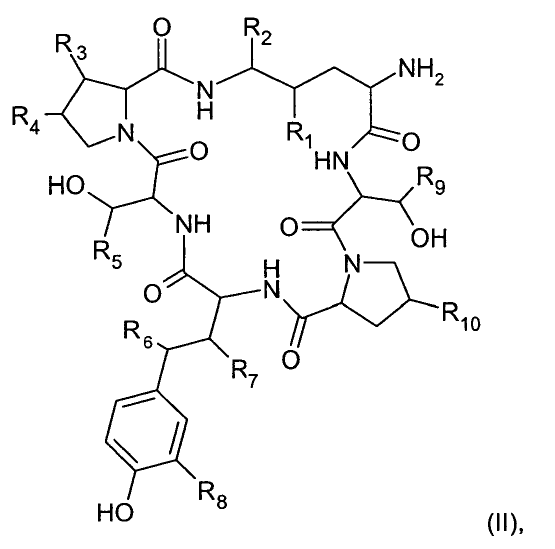 where R₁-R₁₀ have the meaning mentioned for formula (I), and where alkyl, alkenyl, alkadienyl and alkatrienyl groups where present in compounds of the formula (I) and (II) may be branched or straight-chain.

3. The method as claimed in claim 2, where the nucleus (II) is reacted in a further step (e) with an acid derivative to give a semisynthetic lipopeptide of the formula (I') in which R₁-R₁₀ have the meaning mentioned for formula (I) and the group R₁₁ is defined as a C(O)-phenyl-(C₅-C₈)heteroaryl-phenyl, C(O)-biphenyl or C(O)-terphenyl group.

4. The method as claimed in either of claims 2 or 3, where the (C₆-C₂₄)alkyl in the radical R₁₁ of the compound (I) is n-hexyl, n-octyl, n-decanyl, n-undecanyl, n-dodecanyl, n-tridecanyl, n-tetradecanyl, n-pentadecanyl, n-hexadecanyl, n-heptadecanyl, n-octadecanyl, n-nonadecanyl, n-eicosanyl, n-dicosanyl, 9,11-dimethyltridecanyl or 11-methyltridecanyl.

5. The method as claimed in any of claims 2 to 4, where R₁₁ in compound (I) is 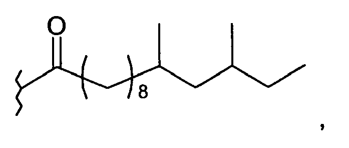 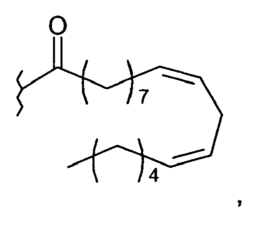 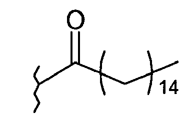 or

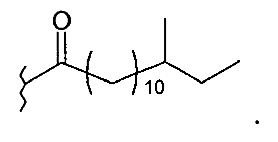

6. The method as claimed in any of claims 2 to 5, where the compound deoxymulundocandin (V) is prepared in step (a) 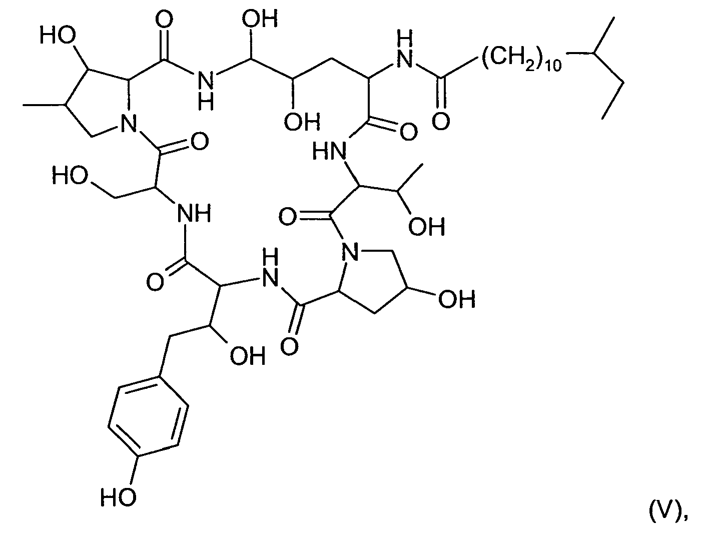 and the deoxymulundocandin nucleus (VI) is formed in step (c)

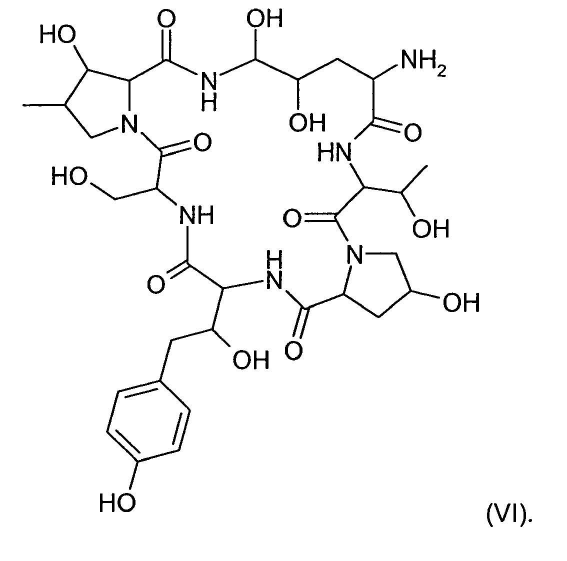

7. The method as claimed in claim 1, where the lipopeptide has the formula (III) 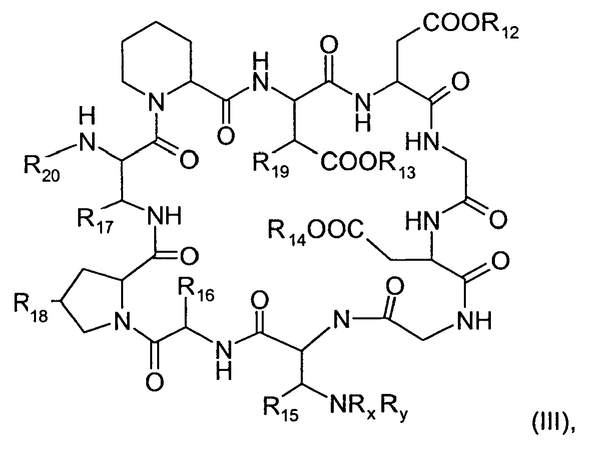 where
R₁₂, R₁₃, R₁₄, R₁₅, R₁₆, R₁₇, R₁₈, R₁₉, Rₓ and R_{y} are independently of one another H or (C₁-C₆)alkyl, and
R₂₀ = a C(O)-CH(CH₂COR₂₁)-NH-CO-(C₆-C₂₄)alkyl group,
a C(O)-CH(CH₂COR₂₁)-NH-CO-(C₆-C₂₄)alkenyl group, a C(O)-CH(CH₂COR₂₁)-NH-CO-(C₆-C₂₄)alkadienyl group, or a C(O)-CH(CH₂COR₂₁)-NH-CO-(C₆-C₂₄)alkatrienyl group, where R₂₁ is OH or NH₂,
to form the corresponding nucleus of the formula (IV) 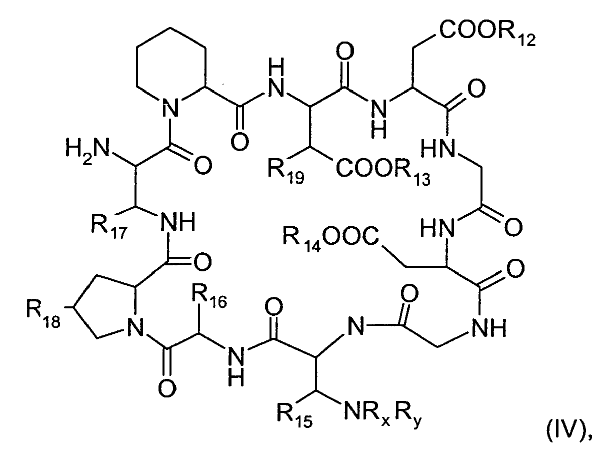 where R₁₂, R₁₃, R₁₄, R₁₅, R₁₆, R₁₇, R₁₈, R₁₉, Rₓ and R_{y} have the meaning mentioned for formula (III), and where alkyl, alkenyl, alkadienyl and alkatrienyl groups where present in compounds of the formula (III) and (IV) may be branched or straight-chain.

8. The method as claimed in claim 7, where R₁₂, R₁₃, R₁₄, R₁₅, R₁₆, R₁₇, R₁₈, R₁₉, Rₓ and R_{y} are equal to H.

9. The method as claimed in either of claims 7 or 8, where R₂₀ is

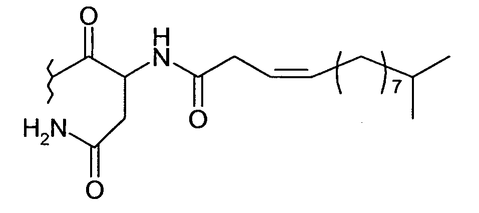

10. The method as claimed in any of claims 7 to 9, where the compound (VII) is prepared in step (a) 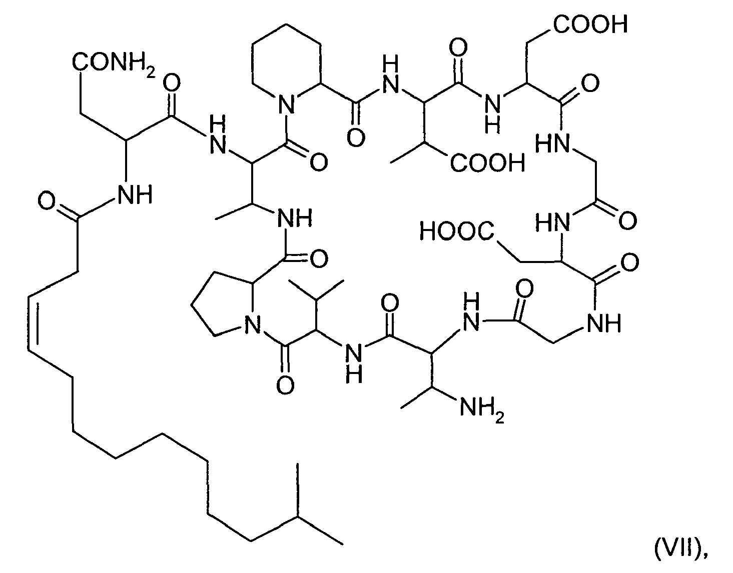 and the compound (VIII) is formed in step (c)

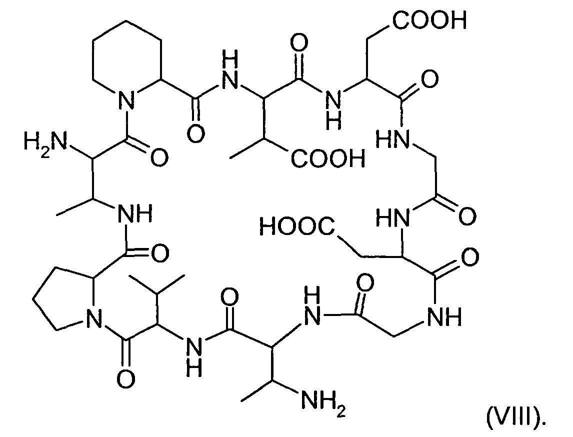

11. The method as claimed in any of claims 1 to 10, where the deacylase has been obtained by cultivating *Actinoplanes utahensis.*

12. The method as claimed in any of claims 1 to 10, where the deacylase is obtained recombinantly by cultivating transformed *Streptomyces lividans.*

## Revendications

1. Procédé pour la séparation enzymatique de la chaîne latérale N-acyle de lipopeptides avec formation du noyau correspondant, dans lequel
(a) le lipopeptide est produit par fermentation, le lipopeptide se trouvant en liaison cellulaire à la biomasse et on sépare la biomasse avec le lipopeptide adhérent,
(b) on remet en suspension dans un système aqueux la biomasse avec le lipopeptide adhérent, provenant de l'étape (a),
(c) on ajoute une désacylase appropriée, sous forme solide ou dissoute, à la suspension de la biomasse provenant de l'étape (b), et il se forme le noyau correspondant, et
(d) éventuellement on isole et purifie le noyau,
**caractérisé en ce que** le lipopeptide obtenu par fermentation dans l'étape (a) est mis directement en réaction dans l'étape (c) avec une désacylase après la fin de la fermentation, sous forme de biomasse liée à la cellule, sans être purifié davantage, de sorte que la chaîne N-acyle reliée par une liaison amide est séparée.

2. Procédé selon la revendication 1, dans lequel le lipopeptide correspond à la formule (I) 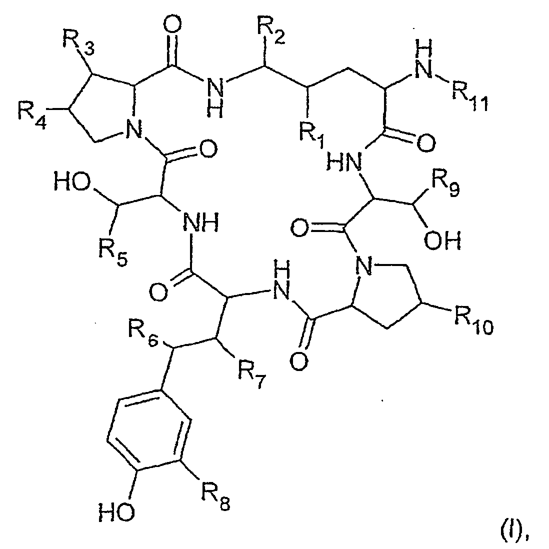 et où
R₁ = H, OH ou NRₓR_{y},
Rₓ et R_{y} représentant, indépendamment l'un de l'autre, H ou un groupe alkyle en C₁-C₆,
R₂ = H, OH, NH(CH₂)₂NH₂,
R₃ = H, OH,
R₄ = H, Me, NH₂, -NH-C(=NH)NH₂,
R₅ = H, Me, CH₂-C(=O)NH₂, CH₂CH₂NH₂,
R₆ = H, OH,
R₇ = H, OH,
R₈ = H, OSO₃H, OSO₃Na, NH-C(=O)CH₂NH₂,
R₉ = Me, et
R₁₀ = H, OH,
R₁₁ = un groupe C(O)-alkyle (C₆-C₂₄), un groupe C(O)-alcényle(C₆-C₂₄), un groupe C(O)-alcadiényle(C₆-C₂₄) ou un groupe C(O)-alcatriényle(C₆-C₂₄),
avec formation du noyau correspondant de formule (II) 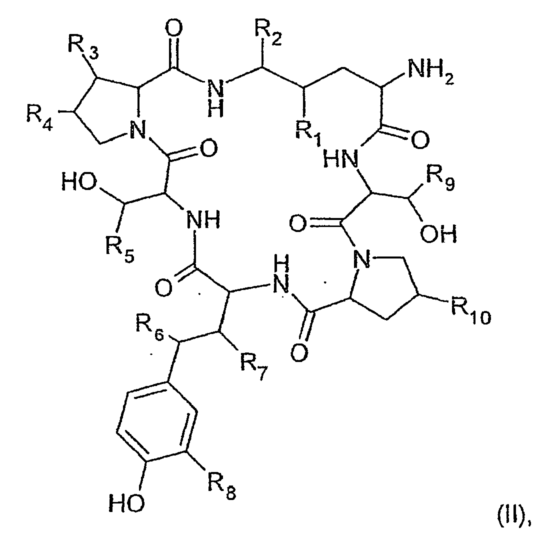 où R₁-R₁₀ ont les significations données pour la formule (I) et où les groupes alkyle, alcényle, alcadiényle et alcatriényle, s'ils sont présents, dans les composés de formules (I) et (II), peuvent être à chaîne droite ou ramifiée.

3. Procédé selon la revendication 2, dans lequel, dans une autre étape (e), on fait réagir le noyau (II) avec un dérivé d'acide, pour aboutir à un lipopeptide semi-synthétique de formule (I') dans lequel R₁-R₁₀ ont les significations données pour la formule (I) et le groupe R₁₁ est défini comme un groupe C(O)-phényl-hétéroaryl(C₅-C₈)-phényle, C(O)-biphényle ou C(O)-terphényle.

4. Procédé selon la revendication 2 ou 3, dans lequel le groupe alkyle en C₆-C₂₄ dans le radical R₁₁ du composé (I) représente le groupe n-hexyle, n-octyle, n-décanyle, n-undécanyle, n-dodécanyle, n-tridécanyle, n-tétradécanyle, n-pentadécanyle, n-hexadécanyle, n-heptadécanyle, n-octadécanyle, n-nonadécanyle, n-eicosanyle, n-dicosanyle, 9,11-diméthyl-tridécanyle ou 11-méthyl-tridécanyle.

5. Procédé selon l'une quelconque des revendications 2 à 4, dans lequel, dans le composé (I), R₁₁ est 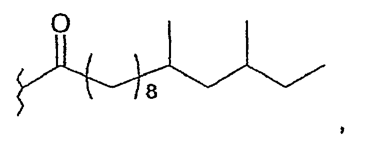 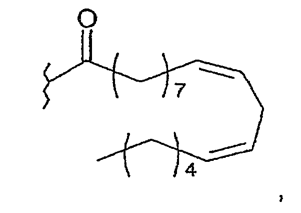 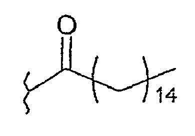 ou

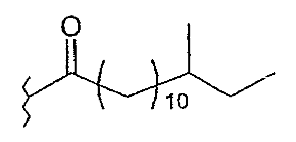

6. Procédé selon l'une quelconque des revendications 2 à 5, dans lequel on prépare dans l'étape (a) le composé désoxymulundocandine (V) 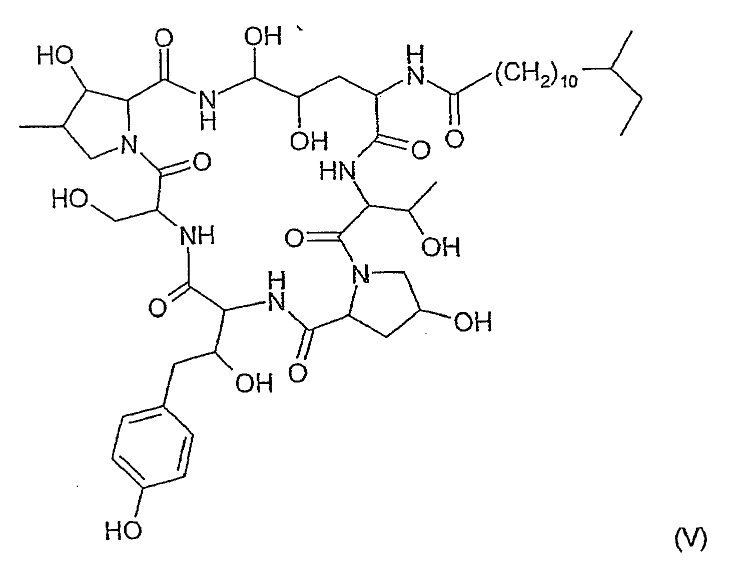 et il se forme dans l'étape (c) le noyau de la désoxymulundocandine (VI)

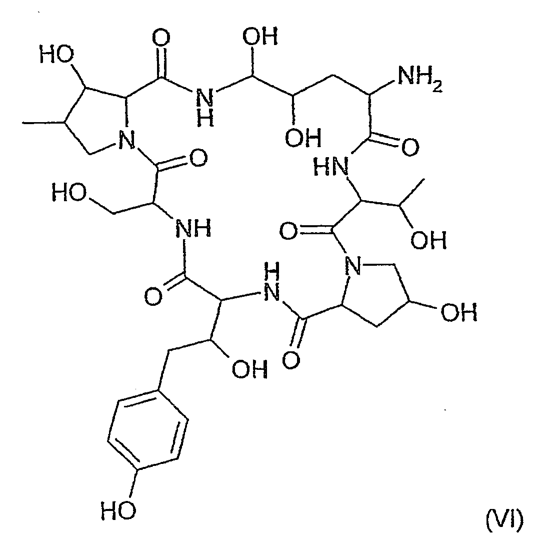

7. Procédé selon la revendication 1, dans lequel le lipopeptide correspond à la formule (III) 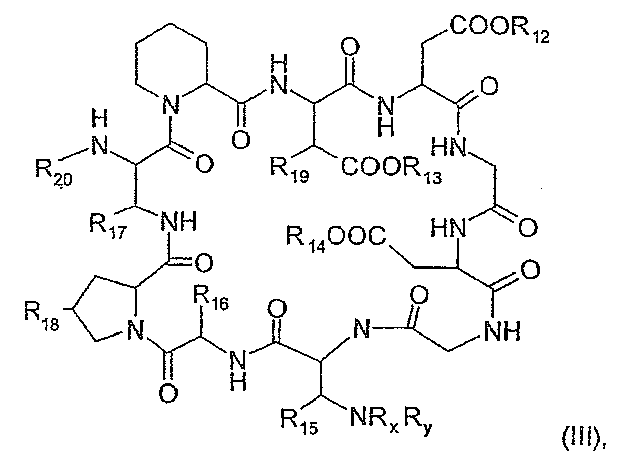 dans laquelle
R₁₂, R₁₃, R₁₄, R₁₅, R₁₆, R₁₇, R₁₈, R₁₉, Rₓ et R_{y} représentent, indépendamment les uns des autres, H ou un groupe alkyle en C₁-C₆, et
R₂₀ représente un groupe C(O)-CH(CH₂COR₂₁)-NH-CO-alkyle(C₆-C₂₄), un groupe C(O)-CH(CH₂COR₂₁)-NH-CO-alcényle(C₆-C₂₄), un groupe C(O)-CH(CH₂COR₂₁)-NH-CO-alcadiényle(C₆-C₂₄), ou un groupe C(O)-CH(CH₂COR₂₁)-NH-CO-alcatriényle(C₆-C₂₄), R₂₁ étant OH ou NH₂,
avec formation du noyau correspondant de formule (IV) 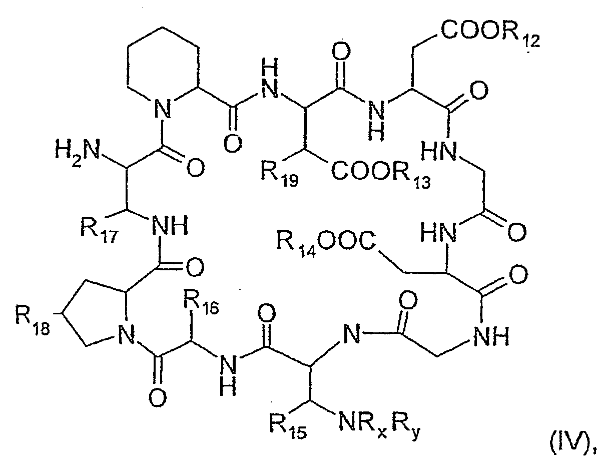 où R₁₂, R₁₃, R₁₄, R₁₅, R₁₆, R₁₇, R₁₈, R₁₉, Rₓ et R_{y} ont les significations données pour la formule (III) et où les groupes alkyle, alcényle, alcadiényle et alcatriényle, s'ils sont présents, dans les composés de formules (III) et (IV), peuvent être à chaîne droite ou ramifiée.

8. Procédé selon la revendication 7, dans lequel R₁₂, R₁₃, R₁₄, R₁₅, R₁₆, R₁₇, R₁₈, R₁₉, Rₓ et R_{y} représentent H.

9. Procédé selon la revendication 7 ou 8, dans lequel R₂₀ est

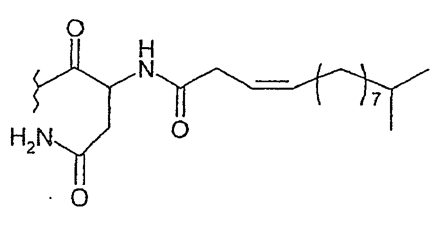

10. Procédé selon l'une quelconque des revendications 7 à 9, dans lequel, dans l'étape (a) on prépare le composé (VII) 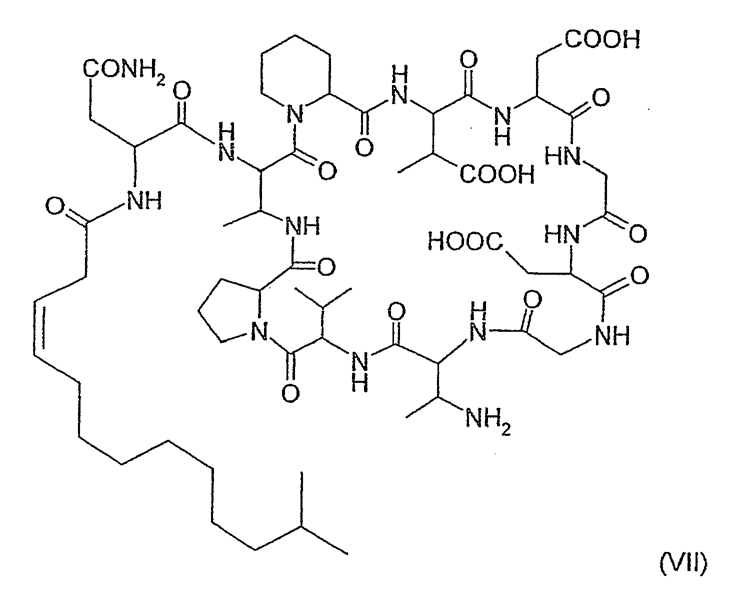 et il se forme dans l'étape (c) le composé (VIII)

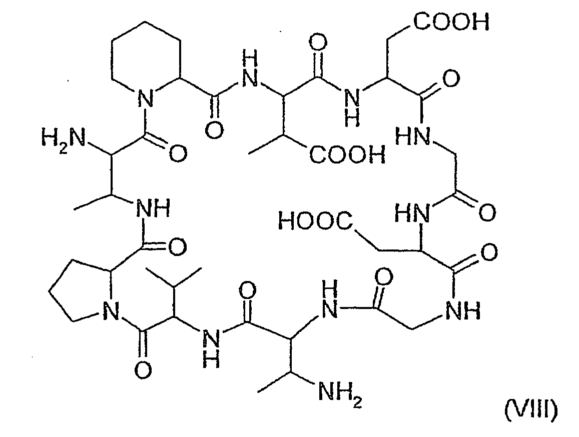

11. Procédé selon l'une quelconque des revendications 1 à 10, dans lequel la désacylase est obtenue par culture d'*Actinoplanes utahensis*.

12. Procédé selon l'une quelconque des revendications 1 à 10, dans lequel la désacylase est obtenue de façon recombinante par culture de *Streptomyces lividans* transformé.
